(19) Europäisches Patentamt / European Patent Office / Office européen des brevets

(11) **EP 4 265 258 A1**

(12) **EUROPEAN PATENT APPLICATION**
published in accordance with Art. 153(4) EPC

(43) Date of publication:
**25.10.2023 Bulletin 2023/43**

(21) Application number: **21906712.1**

(22) Date of filing: **17.12.2021**

(51) International Patent Classification (IPC):
***A61K 31/585*** (2006.01) ***A61K 9/20*** (2006.01)
***A61K 47/10*** (2017.01) ***A61K 47/26*** (2006.01)
***A61K 47/32*** (2006.01) ***A61K 47/36*** (2006.01)
***A61K 47/38*** (2006.01) ***A61P 7/10*** (2006.01)
***A61P 13/02*** (2006.01) ***A61P 13/10*** (2006.01)

(52) Cooperative Patent Classification (CPC):
**A61K 9/20; A61K 31/585; A61K 47/10; A61K 47/26; A61K 47/32; A61K 47/36; A61K 47/38; A61P 7/10; A61P 13/02; A61P 13/10**

(86) International application number:
**PCT/JP2021/046665**

(87) International publication number:
**WO 2022/131354 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **18.12.2020 JP 2020209800**

(71) Applicant: **ASKA Pharmaceutical Co., Ltd.**
**Tokyo 108-8532 (JP)**

(72) Inventors:
- **KITAMURA Sakiko**
  **Fujisawa-shi, Kanagawa 251-8555 (JP)**
- **SASAKI Kazuhiro**
  **Fujisawa-shi, Kanagawa 251-8555 (JP)**
- **KATAOKA Hiroshige**
  **Fujisawa-shi, Kanagawa 251-8555 (JP)**
- **OZAWA Asuka**
  **Fujisawa-shi, Kanagawa 251-8555 (JP)**
- **KOBAYASHI Hideo**
  **Fujisawa-shi, Kanagawa 251-8555 (JP)**
- **SHINBO Atsushi**
  **Fujisawa-shi, Kanagawa 251-8555 (JP)**
- **NAKANO Youichi**
  **Fujisawa-shi, Kanagawa 251-8555 (JP)**
- **ITO Yuta**
  **Fujisawa-shi, Kanagawa 251-8555 (JP)**
- **WATANABE Junichi**
  **Fujisawa-shi, Kanagawa 251-8555 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

# (54) SOLID FORMULATION

(57) Provided is a solid preparation having an excellent stability and being useful for treating or improving (or alleviating) a urination disorder regardless of the degree or presence of prostatomegaly. The solid preparation contains a 2-oxapregnane compound represented by the following formula (1) as an active ingredient, and a carrier.

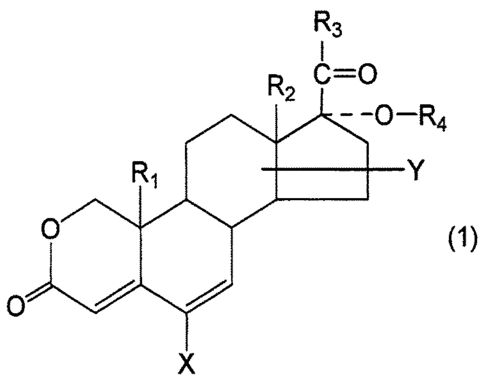

(1)

EP 4 265 258 A1

In the formula, $R_1$ to $R_3$ each represent an alkyl group such as a methyl group, $R_4$ represents an alkylcarbonyl group such as an acetyl group, X represents a halogen atom such as a chlorine atom, and Y represents a hydroxyl group or oxo group bonded to the 11-position, 15-position, or 16-position of the steroid skeleton. The carrier contains a first carrier and/or a second carrier; the first carrier is not a metal salt-form, and the second carrier is a polyvalent metal salt-form of an inorganic acid.

[Fig. 1]

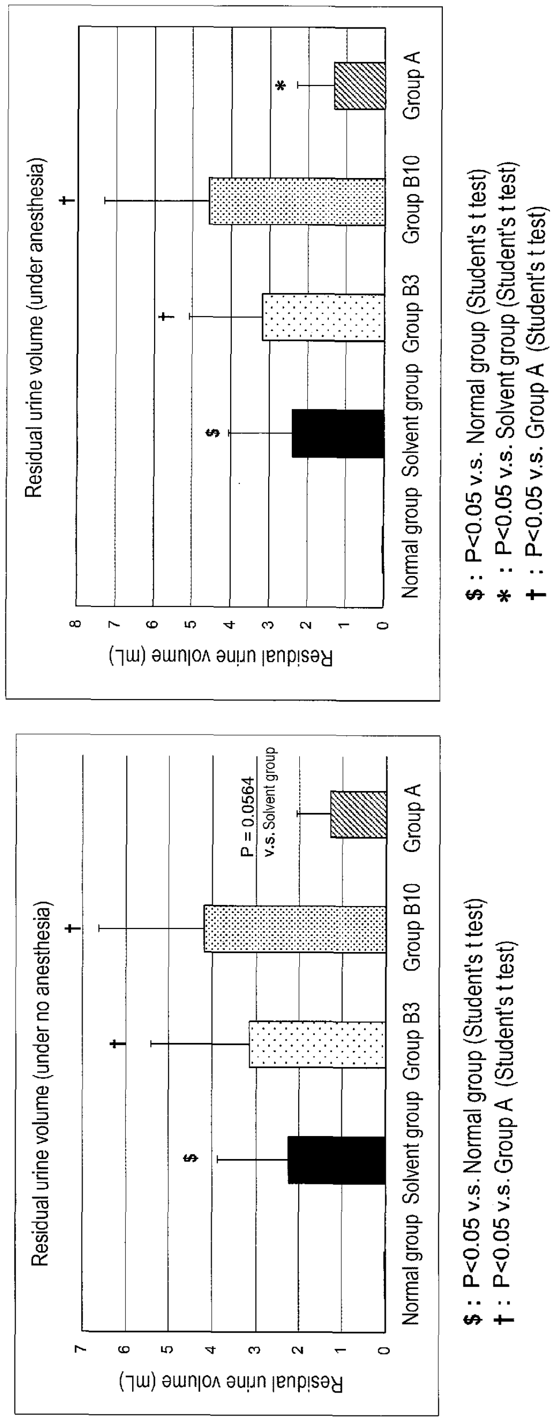

## Description

TECHNICAL FIELD

The present invention relates to a solid preparation (or a urination disorder-improving agent or an agent for improving a urination disorder) containing a pregnane compound or a pharmaceutically acceptable salt thereof as an active ingredient and being useful for improving the urination disorder such as feeling of incomplete emptying.

BACKGROUND ART

As the aging society progresses, it has been desired to improve or alleviate (or mitigate) urination disorders in older persons, where the urination disorders include, for example, diseases or symptoms such as difficulty in urination, feeling of incomplete emptying, frequent urination, and urinary incontinence. As agents for improving such a urination disorder, Japanese Patent Application Laid-Open Publication No. H5-155773 (JP H5-155773 A, Patent Document 1) discloses a xanthine derivative effective in treatment and prevention of a urination disorder such as frequent urination or urinary incontinence. Japanese Patent Application Laid-Open Publication No. H8-3045 (JP H8-3045 A, Patent Document 2) discloses a urination disorder-improving drug containing a N-substituted indole derivative as an active ingredient. WO 2012/020749 (Patent Document 3) discloses a triazine derivative and a pharmaceutical composition containing the triazine derivative and having an analgesic effect or an improving effect of a urination disorder.

Commercially available urination disorder-improving drugs include, for example, an $\alpha$1 blocker and a 5$\alpha$-reductase inhibitor. The 5$\alpha$-reductase inhibitor prevents production of dihydrotestosterone from testosterone and shrinks enlarged prostate, improving a urination disorder.

Japanese Patent Application Laid-Open Publication No. S61-204198 (JP S61-204198 A, Patent Document 4) and Endocrine Journal 1994, 41(4), 445-452 (Nonpatent Document 1) disclose TZP-4238 (17$\alpha$-acetoxy-6-chloro-2-oxa-4,6-pregnadiene-3,20-dione) as an antiandrogenic agent and also disclose that TZP-4238 is useful for treating diseases such as prostatomegaly (or prostate enlargement) and prostatic cancer.

Japanese Patent No. 2591640 (JP 2591640 B, Patent Document 5) discloses a 2-oxapregnane compound as a compound having an antiandrogenic activity, including 17$\alpha$-acetoxy-6-chloro-15$\beta$-hydroxy-2-oxa-4,6-pregnadiene-3,20-dione. This document discloses that the compound is useful as an agent for treating an androgen-dependent disease, for example, benign prostatic hyperplasia, prostatic cancer, and alopecia.

Regarding plant preparations, Japanese Patent Application Laid-Open Publication No. 2011-20972 (JP 2011-20972 A, Patent Document 6) discloses a urination disorder-improving agent containing a saw palmetto extract and containing oleic acid and myristic acid in a ratio of 1:0 to 2:3. The Patent Document 6 discloses that, as males are aged, the prostate enlarges to grow and enlarge the portion (periurethral gland) close to the urethra (mechanical obstruction), and dihydrotestosterone accumulates in the prostate, resulting in a development of benign prostate hyperplasia (BPH). This document discloses that symptoms of benign prostate hyperplasia include lower urinary tract symptoms such as difficulty in urination and frequent urination.

However, the prostate enlargement does not always cause a urination disorder. For example, World Journal of Urology, 13, 9-13 (1995) (Nonpatent Document 2) reports that there is a statistically significant relationship between prostate size and bladder-outlet obstruction while there is no correlation between a symptom of a urination disorder and a degree of bladder-outlet obstruction or a prostate size.

The Journal of Urology, 150, 351-358 August 1993 (Nonpatent Document 3) reports that there is no correlation between prostate size and residual urine volume and that there is no correlation between residual urine volume or prostate size and various symptoms. Specifically, this document provides a correlation matrix for patients with benign prostatic hyperplasia (BPH) and discloses that, despite a relatively large sample size, there were no statistically significant relationships between symptom severity and residual urine volume, prostate size or prostate specific antigen (PSA) level. This document discloses that prostate enlargement, which is also an attribute of BPH, is not a critical factor in the production of either symptoms or physiological outlet obstruction. In addition, this document discloses that in some patients (individuals), a physiological disorder can be induced even if the degree of hyperplasia in the periurethral area is small, while in other patients (individuals), considerable hyperplasia can occur without developing the disorder.

Thus, it is intended to effectively treat or improve a urination disorder regardless of the degree or presence of prostate enlargement. Further, the treatment of the urination disorder includes administration to patients such as older persons for a long period of time and is to have a high therapeutic effect and a high safety.

CITATION LIST

PATENT LITERATURE

Patent Document 1: JP H5-155773 A (Claims)
Patent Document 2: JP H8-3045 A (Claims)
Patent Document 3: WO 2012/020749 (Claims)
Patent Document 4: JP S61-204198 A (Claims, and page 1, the right column)
Patent Document 5: JP 2591640 B (Claims, and column 11, line 49 to column 12, line 49)
Patent Document 6: JP 2011-20972 A (Claims and [0002])

NONPATENT LITERATURE

Nonpatent Document 1: Endocrine Journal 1994, 41(4), 445-452
Nonpatent Document 2: World Journal of Urology, 13, 9-13 (1995) (Summary)
Nonpatent Document 3: The Journal of Urology, 150, 351-358 August 1993 (page 354, the right column, lines 5 to 10, page 355, the left column, line 9 from the bottom to page 355, the right column, line 6, Table 3, and Table 5)

SUMMARY OF INVENTION

TECHNICAL PROBLEM

It is therefore an object of the present invention to provide a solid preparation (or a urination disorder-improving agent) useful for treating or improving (or alleviating or mitigating) the urination disorder.

Another object of the present invention is to provide a solid preparation (or a urination disorder-improving agent) having an excellent stability without discoloration or coloration over a long period of time.

It is another object of the present invention to provide a solid preparation (or a urination disorder-improving agent) having a high safety and being useful for treating or improving the urination disorder with a high therapeutic or improving effect even if the preparation is administered for a long period of time.

It is still another object of the present invention to provide a solid preparation (or a urination disorder-improving agent) useful for treating or alleviating (or mitigating) the urination disorder regardless of the degree or presence of prostatomegaly (or prostate enlargement).

SOLUTION TO PROBLEM

The inventors of the present invention made intensive studies on a relationship between a urination disorder and 2-oxapregnane compounds having an oxo group on the 2-position of a steroid skeleton thereof and having a high antiandrogenic activity to achieve the above objects and found that even if the 2-oxapregnane compounds have a high antiandrogenic activity, great variations in improvement of the urination disorder are found depending on the presence or absence of a hydroxyl group or oxo group on a predetermined site of a steroid skeleton thereof. Specifically, the inventors found the following: a compound without a hydroxyl group on the predetermined site of the steroid skeleton shows no therapeutic effects on the urination disorder in a subject having no prostatomegaly, in spite of a high antiandrogenic activity thereof, while a compound with a hydroxyl group or oxo group on the predetermined site of the steroid skeleton shows a high therapeutic or improving effect on the urination disorder regardless of the presence of prostatomegaly. Further, the inventors found that, in a solid preparation containing the 2-oxapregnane compound, a carrier in a metal salt-form of an organic acid produces discoloration or coloration of the solid preparation. The present invention was accomplished based on the above findings.

That is, the present invention provides a solid preparation or sold formulation [or a urination disorder-improving agent (a urination disorder-alleviating agent, or an agent for improving or alleviating a urination disorder)] at least containing (a) an active ingredient and (b) a carrier; the active ingredient (a) contains a 2-oxapregnane compound represented by the following formula (1) or a pharmaceutically acceptable salt thereof, and the carrier (b) contains a first carrier and/or a second carrier; the first carrier is not a metal salt-form, and the second carrier is a polyvalent metal salt-form of an inorganic acid. Incidentally, in the following formula (1), respective positions of the steroid skeleton are denoted by positional numbers.

[Chem. 1]

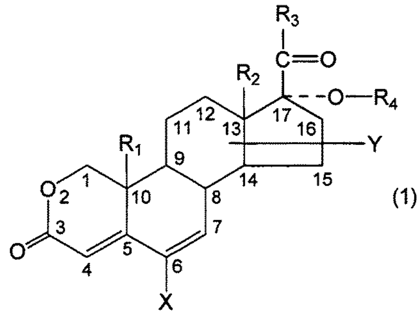

(1)

In the formula, $R_1$ to $R_3$ are the same or different and each represent an alkyl group, $R_4$ represents a hydrogen atom or an alkylcarbonyl group, X represents a halogen atom, and Y represents a hydroxyl group or oxo group bonded to the 11-position, 15-position, or 16-position of the steroid skeleton.

$R_1$ to $R_3$ are the same or different and may each represent a $C_{1-4}$alkyl group (for example, a methyl group). $R_4$ may represent a lower alkanoyl group or acyl group (a $C_{1-4}$alkyl-carbonyl group), for example, an acetyl group. The halogen atom represented by X may be a chlorine atom. Y may represent a hydroxyl group or oxo group bonded to the 15-position of the steroid skeleton. A representative 2-oxapregnane compound represented by the formula (1) may be 17α-acetoxy-6-chloro-15β-hydroxy-2-oxa-4,6-pregnadiene-3,20-dione.

The carrier (b) may be at least one member selected from (b-1) a diluent or excipient, (b-2) a binder, and (b-3) a disintegrator.

The diluent (b-1) may be at least one member selected from a saccharide or a sugar alcohol, a starch, a polysaccharide, and an alkaline earth metal salt of an inorganic acid. Moreover, the diluent (b-1) may be at least one member selected from a lactose, a corn starch, a crystalline cellulose, and a calcium phosphate.

The disintegrator (b-3) may be at least one member selected from a pregelatinized starch, a crospovidone, and a low substituted hydroxypropylcellulose.

The solid preparation according to the present invention may further contain a lubricant.

In the solid preparation according to the present invention, the carrier (b) contains the first carrier, which is not a metal salt-form, and/or the second carrier, which is a polyvalent metal salt-form of an inorganic acid, and thus the present invention allows the effective prevention of discoloration or coloration of the solid preparation.

The solid preparation according to the present invention may contain a metal salt of an organic acid (a metal salt of an organic compound having an acid group) in the carrier (b) in a ratio of 10% by mass or less (that is, 0 to 10% by mass) in terms of metal. Specifically, the solid preparation can effectively be prevented from discoloration or coloration in a case where the solid preparation is substantially free from a carrier in a metal salt-form of an organic acid, and the solid preparation can effectively be prevented from discoloration or coloration even if the solid preparation contains a carrier in a metal salt-form of an organic acid, in a case where the metal content of the carrier is 10% by mass or less (that is, 0 to 10% by mass) in terms of metal.

The solid preparation according to the present invention is useful for preventing, treating, or improving or alleviating (or mitigating) a urination disorder or symptom. For example, the urination disorder or symptom may be at least one urination disorder (or disease) or symptom selected from feeling of incomplete emptying, slow stream, hesitancy (or urination delay), straining (or abdominal pressure urination), terminal dribble, post micturition dribble, urinary incontinence (including urge, stress, mixed, and overflow), frequent urination (including increased daytime frequency, nocturia, and psychogenic frequent urination), urgency, bladder pain or bladder pain syndrome, pain on urination, cystatrophia, neurogenic bladder, detrusor overactivity or overactive bladder (including idiopathic, neurogenic, and refractory), chronic cystitis, interstitial cystitis, chronic prostatitis, pelvic pain syndrome, underactive bladder, and detrusor hyperactivity with impaired contractile function (or detrusor hyperactivity with impaired contraction). Moreover, the solid preparation according to the present invention is useful for improving at least one urination disorder or symptom selected from residual urine volume, urination efficiency, bladder capacity, bladder wet weight, and kidney wet weight. Specifically, the solid preparation according to the present invention shows a high therapeutic or improving effect on the urination disorder regardless of the presence of benign prostatic hyperplasia. That is, the urination disorder may be either a urination disorder having no benign prostatic hyperplasia or a urination disorder associated with benign prostatic hyperplasia. Thus, a subject with the urination disorder may be either a male or a female (a man or a woman). The urination disorder may be a urination disorder associated with a smooth muscle contraction disorder. The urination disorder may be a urination disorder associated with at least one disease selected from the following: a disease causing or inducing neurogenic bladder; diabetes; and neuropathy associated with diabetes. The solid preparation (or the urination disorder-improving agent) may improve or promote urination. Thus, the solid preparation (or the urination disorder-improving

agent) may also be referred to as a urination improver or a urination promotor.

The present invention includes a method for treating, or improving or alleviating (or mitigating) a urination disorder (and a method for improving or promoting urination), the method comprising administering, to a subject with a urination disorder, a 2-oxapregnane compound represented by the formula (1) or a pharmaceutically acceptable salt thereof as an active ingredient. Further, the present invention includes use of a 2-oxapregnane compound represented by the formula (1) or a pharmaceutically acceptable salt thereof as an active ingredient for treating, or improving or alleviating (or mitigating) a urination disorder (and improving or promoting urination); and use of a 2-oxapregnane compound represented by the formula (1) or a pharmaceutically acceptable salt thereof for producing the urination disorder-improving agent, the urination improver, or the urination promotor. Furthermore, the present invention includes a method for improving the stability (or a method for preventing discoloration or coloration) of a 2-oxapregnane compound represented by the formula (1) or a pharmaceutically acceptable salt thereof, or a solid preparation, the method comprising adding (b) a carrier containing the first carrier and/or the second carrier to the 2-oxapregnane compound or the pharmaceutically acceptable salt thereof. In the method for improving the stability of the solid preparation, the proportion of the metal salt of the organic acid in the carrier (b) may be 10% by mass or less in terms of metal (that is, 0 to 10% by mass), specifically, the carrier may be a carrier that is not a metal salt-form or may contain a metal salt (a metal salt of an organic acid) at a predetermined low metal content. The dosage for a human being as a subject (or patient) may be about 0.01 to 100 mg per day in terms of the compound represented by the formula (1).

In this description and claims, an organic compound having an acid group may be simply referred to as an "organic acid". In this description and claims, the expression "the metal proportion (or metal content) is 0%" means that metals unavoidably mixed may be included (unavoidable metals are allowed).

In this description and claims, a lubricant is not included in the concept of a carrier and is included in an additive rather than a carrier (or classified as an additive).

In this description and claims, a carrier that is not a metal salt-form (a first carrier) means not to contain a carrier (a third carrier) that is a metal salt of an organic acid or a carrier (a fourth carrier) that is an alkali metal salt of an inorganic acid (and an alkali metal halide).

In this description and claims, the discoloration or coloration of a solid preparation can be evaluated as follows: a mixture (as a composition for the solid preparation) is stored under constant temperature and humidity conditions (a temperature of 40°C and a relative humidity of 75%) for a predetermined period (4 weeks, preferably 6 weeks), and a change (difference or increase) from a color difference ($\Delta E^*$) of the initial mixture to a color difference ($\Delta E^*$) of the mixture after the storing is determined, where the color difference ($\Delta E^*$) is measured using a $L^*a^*b^*$ colorimeter (a spectrophotometer). According to this evaluation method, a color difference ($\Delta E^*$) of not less than 3 [an increase or decrease in color difference ($\Delta E^*$) by not less than 3] allows the confirmation of the discoloration or coloration by visual observation.

## ADVANTAGEOUS EFFECTS OF INVENTION

According to the present invention, the solid preparation (or the urination disorder-improving agent) contains the specific 2-oxapregnane compound or the pharmaceutically acceptable salt thereof and thus enables the effective treatment or improvement (or alleviation or mitigation) of the urination disorder. Moreover, according to the present invention, the solid preparation (or the urination disorder-improving agent) has an excellent stability without discoloration or coloration even when stored over a long period of time. Further, the compound or the pharmaceutically acceptable salt thereof has a high safety, allowing the urination disorder to be improved with a high therapeutic or improving effect even when administered for a long period of time. Furthermore, the compound or the pharmaceutically acceptable salt thereof has a hydroxyl group or oxo group on the predetermined site of the steroid skeleton and thus enables the treatment or alleviation (or mitigation) of the urination disorder regardless of the degree or presence of prostatomegaly.

## BRIEF DESCRIPTION OF DRAWINGS

[Fig. 1] Fig. 1 is a graph showing a relationship between the residual urine volume and the test substances under no anesthesia and under anesthesia in Test Example 1.

[Fig. 2] Fig. 2 is a graph showing a relationship between the urination efficiency and the test substances under no anesthesia and under anesthesia in Test Example 1.

[Fig. 3] Fig. 3 is a graph showing a relationship between the bladder capacity and the test substances in Test Example 1.

[Fig. 4] Fig. 4 is a graph showing a relationship between the bladder wet weight and the test substances in Test Example 1.

[Fig. 5] Fig. 5 is a graph showing a relationship between the kidney wet weight and the test substances in Test

Example 1.

[Fig. 6] Fig. 6 is a graph showing a relationship between the survival rate and the test substances in Test Example 1.

[Fig. 7] Fig. 7 is a graph showing a relationship between the residual urine volume and the test substances in Test Example 2.

[Fig. 8] Fig. 8 is a graph showing a relationship between the urination efficiency and the test substances in Test Example 2.

[Fig. 9] Fig. 9 is a graph showing a relationship between the bladder contraction tension by square-wave electrical field stimulation and the test substances in Test Example 3.

[Fig. 10] Fig. 10 is a graph showing a relationship between the test substances and the bladder contraction tension by Carbachol (muscarinic receptor stimulation) and KCl (depolarization-dependent $Ca^{2+}$ stimulation) in Test Example 3.

[Fig. 11] Fig. 11 is a graph showing a relationship between the bladder capacity and the test substances in cystometry (CMG) under awake condition of Test Example 4.

[Fig. 12] Fig. 12 is a graph showing a relationship between the residual urine volume and the test substances in cystometry (CMG) under awake condition of Test Example 4.

[Fig. 13] Fig. 13 is a graph showing a relationship between the urination efficiency and the test substances in cystometry (CMG) under awake condition of Test Example 4.

[Fig. 14] Fig. 14 is a graph showing a relationship between the bladder capacity and the test substances in cystometry (CMG) under anesthesia of Test Example 4.

[Fig. 15] Fig. 15 is a graph showing a relationship between the residual urine volume and the test substances in cystometry (CMG) under anesthesia of Test Example 4.

[Fig. 16] Fig. 16 is a graph showing a relationship between the urination efficiency and the test substances in cystometry (CMG) under anesthesia of Test Example 4.

[Fig. 17] Fig. 17 is a graph showing the results of nomogram analysis in Test Example 4.

[Fig. 18] Fig. 18 is a graph showing a relationship between the uterine weight and the test substances in Test Example 5.

[Fig. 19] Fig. 19 is a graph showing a relationship between the residual urine volume and the test substances in cystometry (CMG) under awake condition of Test Example 6.

[Fig. 20] Fig. 20 is a graph showing a relationship between the urination efficiency and the test substances in cystometry (CMG) under awake condition of Test Example 6.

[Fig. 21] Fig. 21 is a graph showing the results of nomogram analysis in Test Example 6.

[Fig. 22] Fig. 22 is a graph showing evaluation results of the color difference (ΔE*) in Examples 1 to 4 and Reference Examples 4 to 6.

[Fig. 23] Fig. 23 is a graph showing evaluation results of the color difference (ΔE*) in Examples 5 to 7 and Reference Examples 1 to 3.

[Fig. 24] Fig. 24 is a graph showing evaluation results of the color difference (ΔE*) in the lactose hydrate of Example 9.

[Fig. 25] Fig. 25 is a graph showing evaluation results of the color difference (ΔE*) in the crystalline cellulose of Example 9.

[Fig. 26] Fig. 26 is a graph showing a relationship between the type and mixed amount of the disintegrator, and the color difference (ΔE*) in Example 10.

DESCRIPTION OF EMBODIMENTS

(a) Active ingredient [2-Oxapregnane compound or pharmaceutically acceptable salt thereof]

A compound represented by the formula (1) is described in the Patent Document 5 and has been known. As mentioned above, such a compound has a high antiandrogenic activity and is useful as an agent for treating benign prostatic hyperplasia, while the compound has a characteristic that effectively treats or improves a urination disorder regardless of prostate enlargement.

In the formula (1), as examples of an alkyl group represented by each of $R_1$ to $R_3$, there may be mentioned a straight- or branched-chain $C_{1-6}$alkyl group such as methyl group, ethyl group, propyl group, isopropyl group, butyl group, isobutyl group, s-butyl group, and t-butyl group. The alkyl group is usually a straight- or branched-chain $C_{1-4}$alkyl group, preferably a straight- or branched-chain $C_{1-3}$alkyl group, and further preferably methyl group or ethyl group. The alkyl group may practically be methyl group. Specifically, $R_1$ to $R_3$ may be the same or a different alkyl group. Each of $R_1$ to $R_3$ may practically be methyl group.

$R_4$ may be either a hydrogen atom or an alkylcarbonyl group and may practically be an alkylcarbonyl group (an alkanoyl group or acyl group). The alkylcarbonyl group (or acyl group) represented by $R_4$ may include, for example, a straight- or branched-chain $C_{1-10}$alkyl-carbonyl group such as acetyl group, propionyl group, butyryl group, isobutyryl

group, t-butyryl group, pentanoyl group (valeryl group), and hexanoyl group. The alkylcarbonyl group is usually a straight- or branched-chain $C_{1-6}$alkyl-carbonyl group, preferably a $C_{1-4}$alkyl-carbonyl group, and further preferably a $C_{1-3}$alkyl-carbonyl group. The alkylcarbonyl group may practically be acetyl group. The alkylcarbonyl group represented by $R_4$ may be converted into a hydrogen atom by hydrolysis with a hydrolase. The hydrogen atom represented by $R_4$ may be converted into an alkylcarbonyl group by an acylase.

A halogen atom represented by X may include a fluorine atom, a chlorine atom, a bromine atom, and an iodine atom. The halogen atom may practically be a fluorine atom, a chlorine atom, or a bromine atom, particularly a chlorine atom.

The substitution position of a hydroxyl group or oxo group represented by Y may be either the 11-position, 15-position, or 16-position of the steroid skeleton. The hydroxyl group or oxo group may practically be bonded to the 15-position of the steroid skeleton.

Oxo groups and hydroxyl groups of the steroid skeleton may mutually be convertible by an in-vivo oxidoreductase. Thus, Y may be either a hydroxyl group or an oxo group. Y may practically be a hydroxyl group. In a case where Y is a hydroxyl group, the conformation of the hydroxyl group may be the α-position or β-position represented by the following formulae. For example, the conformation of the hydroxyl group bonded to the 15-position of the steroid skeleton may be the β-position.

[Chem. 2]

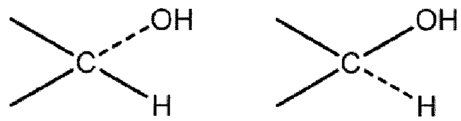

α-position β-position

The compound represented by the formula (1) may include a 17α-$C_{1-4}$alkyl-carbonyloxy-6-halo-15β-hydroxy-2-oxa-4,6-pregnadiene-3,20-dione and a 17α-$C_{1-4}$alkyl-carbonyloxy-6-halo-2-oxa-4,6-pregnadiene-3,15,20-trione.

A preferred compound may have the hydroxyl group represented by Y on the 11-position, 15-position, or 16-position of the steroid skeleton and can be represented by the following formula (1a).

[Chem. 3]

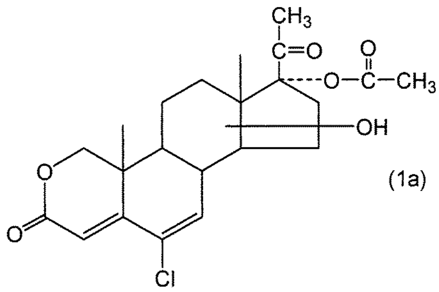

(1a)

Representative examples of the compound represented by the formula (1a) or the pharmaceutically acceptable salt thereof may include 17α-acetoxy-6-chloro-15β-hydroxy-2-oxa-4,6-pregnadiene-3,20-dione or a pharmaceutically acceptable salt thereof.

The 2-oxapregnane compound represented by the formula (1) or the pharmaceutically acceptable salt thereof may be a racemate or may be an optically active substance (or an optical isomer). The 2-oxapregnane compound represented by the formula (1) or the pharmaceutically acceptable salt thereof may be a metabolite.

Examples of the salt may include a salt of an inorganic acid (such as hydrochloric acid and sulfuric acid), a salt of an organic acid (such as acetic acid), a salt of an inorganic base (e.g., ammonia, an alkali metal such as sodium and potassium, and an alkaline earth metal such as calcium), and a salt of an organic base (e.g., amines such as triethylamine).

The 2-oxapregnane compound represented by the formula (1) or the pharmaceutically acceptable salt thereof can be prepared according to a conventional method, for example, a method described in the Patent Document 5.

The 2-oxapregnane compound represented by the formula (1) or the pharmaceutically acceptable salt thereof may have an antiandrogenic activity or may act as an androgen receptor modulator. Further, the compound may have

both an antiandrogenic action and an antiprogesterone action.

The urination disorder-improving agent according to the present invention contains at least the 2-oxapregnane compound represented by the formula (1) or the pharmaceutically acceptable salt thereof as (a) an active ingredient and, if necessary, may further contain a physiologically active ingredient or a pharmacologically active ingredient (such as another urination improver). Examples of another urination improver (a second urination improver) may include an $\alpha$1 receptor blocker (a depressor) or a prostatomegaly-treating drug (such as doxazosin mesilate, urapidil, prazosin hydrochloride, bunazosin hydrochloride, oxendolone, gestonorone caproate, chlormadinone acetate, tamsulosin hydrochloride, naftopidil, and silodosin), a 5$\alpha$ reductase inhibitor (such as Dutasteride), an antiandrogen drug (such as methyltestosterone, testosterone propionate, testosterone enanthate, bicalutamide, and enzalutamide), a PDE5 inhibitor (such as tadalafil), and a crude drug. Such a second urination improver may be used in a ratio of about 0.1 to 100 parts by mass (e.g., about 1 to 50 parts by mass) relative to 100 parts by mass of the 2-oxapregnane compound represented by the formula (1).

The urination disorder-improving agent (or preparation) according to the present invention is effective in treating and/or preventing various urination disorders and improves or alleviates (or mitigates) the urination disorders. Thus, the urination disorder-improving agent may also be referred to as a therapeutic agent and/or preventive agent for treating and/or preventing a urination disorder or an alleviator or mitigator for alleviating or mitigating a urination disorder.

Examples of the urination disorder may include a disease or symptom such as difficulty in urination (disturbance on urination), feeling of incomplete emptying, slow stream, hesitancy, straining, terminal dribble, post micturition dribble, urinary incontinence (including urge, stress, mixed, and overflow), frequent urination (including increased daytime frequency, nocturia, and psychogenic frequent urination), urgency, bladder pain or bladder pain syndrome, pain on urination, cystatrophia, neurogenic bladder, detrusor overactivity or overactive bladder (including idiopathic, neurogenic, and refractory), chronic cystitis, interstitial cystitis, chronic prostatitis, pelvic pain syndrome, underactive bladder, and detrusor hyperactivity with impaired contractile function. Further, the symptom may be at least one symptom selected from increased residual urine volume, reduced urination efficiency, increased bladder capacity, and increased bladder wet weight and kidney wet weight. Such at least one symptom may be used as an index or criterion of the urination disorder. The urination disorder may be a disorder that causes these plural diseases or symptoms complexly. For example, difficulty in urination may be accompanied with feeling of incomplete emptying and slow stream. The urination disorder may practically occur as a symptom (for example, a lower urinary tract symptom) including feeling of incomplete emptying, slow stream, urinary incontinence, frequent urination, or urgency. Thus, according to the present invention, urination may be improved or promoted.

The above-mentioned urination disorder may be accompanied with prostatomegaly or may not be accompanied with prostatomegaly. That is, the urination disorder may be either a urination disorder having no benign prostatic hyperplasia or a urination disorder associated with benign prostatic hyperplasia. The present invention enables the urination disorder to be effectively treated or improved regardless of the degree and/or presence of prostatomegaly.

The urination disorder may be a urination disorder associated with a smooth muscle contraction disorder (a bladder smooth muscle contraction disorder). The types of the urination disorder may include, for example, urination disorders associated with the following diseases: a disease causing or inducing neurogenic bladder; diabetes; neuropathy associated with diabetes (afferent or efferent neuropathy of the micturition reflex system); hyperglycemia; and neuropathy associated with hyperglycemia. These urination disorders may be accompanied with benign prostatic hyperplasia or may not be accompanied with benign prostatic hyperplasia, and may practically be a urination disorder having no benign prostatic hyperplasia. The disease causing or inducing neurogenic bladder may be, for example, cerebrovascular disorder (stroke), Alzheimer's disease, Parkinson's disease, multiple sclerosis, cerebellar degeneration, myelomeningocele (spina bifida), tethered cord syndrome, herniated intervertebral disc, spinal stenosis, and peripheral neuropathy to the bladder caused by rectal cancer or uterine cancer surgery. Concretely, in neurogenic bladder as an example, even if no benign prostatic hyperplasia is accompanied, a urination disorder may be caused by inhibition of smooth muscle contraction due to central or peripheral neuropathy. Moreover, it is known that, in diabetes patients and/or hyperglycemia patients regardless of insulin dependence or insulin independence, the central or peripheral nerve is impaired or injured to cause a urination disorder due to complication neuropathy (such as sensory (afferent) or efferent neuropathy of the micturition reflex system). The diabetes may be type I and/or type II diabetes. Thus, the urination disorder may be caused by the inhibition of bladder smooth muscle contraction through the neuropathy. Moreover, clinically and nonclinically, it is also known that the urination disorder (for example, underactive bladder, a diabetic urination disorder) is associated with a bladder smooth muscle contraction disorder. The present invention enables such a urination disorder having no benign prostatic hyperplasia to be effectively treated or improved. Thus, a subject with the urination disorder may be either a male or a female (a man or a woman). The compound or the pharmaceutically acceptable salt thereof and the urination disorder-improving agent (or preparation) according to the present invention are useful for effectively treating or improving the urination disorder regardless of sex.

The solid preparation (or the urination disorder-improving agent) according to the present invention may contain (a) the 2-oxapregnane compound represented by the formula (1) or the pharmaceutically acceptable salt thereof as the

active ingredient, and (b) the carrier (such as a pharmacologically or physiologically acceptable carrier). The carrier of the urination disorder-improving agent (the pharmaceutical composition, the physiologically active composition) may be selected depending on the form (that is, the dosage form) of the preparation, the route of administration, the application (or use), and others. The solid preparation may have a dosage form of powdered preparations, powders, granulated preparations (such as granules and fine granules), spherical or spheroidal preparations, pills, tablets, capsules (such as soft capsules and hard capsules), dry syrups, suppositories, and film- or sheet-like preparations. In a preferred embodiment, the solid preparation according to the present invention particularly has a dosage form of granulated preparations, tablets, or capsules.

The powdered preparations may also include sprays, aerosols, and others. The capsules may be either soft capsules or hard capsules, or may be a capsule filled with a solid dosage form such as granules. Moreover, the preparation may be a freeze-dried preparation. Further, the urination disorder-improving agent (or the solid preparation) according to the present invention may be a preparation having a controlled release rate of a pharmaceutical component (an extended-release or sustained-release preparation or a rapid-release preparation). Moreover, the administration route of the urination disorder-improving agent (or the solid preparation) is not limited to a specific route. The urination disorder-improving agent (or the solid preparation) may be an orally administrable preparation [for example, granules, powders, tablets (e.g., sublingual tablets and orally disintegrating tablets), capsules, and film preparations] or a parenterally administrable preparation (for example, inhalations, preparations for transdermal administration, preparations for transnasal administration, and suppositories). Furthermore, the preparation may be a topically or locally administrable preparation. A preferred solid preparation includes, for example, an orally administrable solid preparation.

(b) Carrier

The carrier for the solid preparation may be selected, depending on the administration route and the application of preparation, from components (e.g., diluents or excipients, binders, and disintegrators) listed in, for example, The Japanese Pharmacopoeia Eighteenth Edition, (1) Handbook of Pharmaceutical Excipients, International Pharmaceutical Excipients Council Japan (2007), (2) Japanese Pharmaceutical Excipients Dictionary 2016 (Yakuji Nippo Ltd., issued February, 2016), (3) Pharmaceutics, revised fifth edition (Nankodo, Co., Ltd. (1997)), and (4) Japanese Pharmaceutical Excipients 2018 (Yakuji Nippo Ltd., issued July, 2018). For example, the carrier practically used for a solid preparation includes at least one carrier selected from (b-1) a diluent and (b-3) a disintegrator, or at least one carrier selected from (b-1) a diluent, (b-2) a binder, and (b-3) a disintegrator. In particular, it is preferred that the carrier for the solid preparation contain the diluent (b-1) and at least the disintegrator (b-3) among the binder (b-2) and the disintegrator (b-3). To the carrier may further be added a lubricant. Moreover, the solid preparation may be coated with a coating agent. The solid preparation may contain a lipid.

The metal salt may include, for example, a metal salt in a form of a salt of an inorganic acid, such as a sulfate, a hydrochloride, a phosphate, a carbonate, and a silicate; and a metal salt in a form of a salt of an organic acid, such as a carboxylate and a sulfonate. The metal salt preferably includes a metal salt in a form of a salt of an inorganic acid, such as a sulfate, a phosphate, a carbonate, and a silicate, or a metal salt in a form of a carboxylate (in particular, a metal salt in a form of a carboxylate). Examples of the metal salt may include an alkali metal salt such as a sodium salt and a potassium salt; and a polyvalent metal salt including an alkaline earth metal salt (or a divalent metal salt) such as a calcium salt and a magnesium salt, and a trivalent metal salt (for example, an aluminum salt). Such a carrier may be a carrier having a free acid group such as a carboxyl group and a sulfonic acid group (particularly, a carboxyl group) or may be a carrier free from an acid group. The alkali metal salt probably has an influence on coloration or discoloration compared with the polyvalent metal salt such as the alkaline earth metal salt, and thus the polyvalent metal salt such as the alkaline earth metal salt is preferably used from the point of view that the solid preparation has less or no coloration or less or no discoloration.

Specifically, a water-soluble metal salt has a large influence on the coloration or discoloration of the solid preparation, and a water-swelling (gel-forming or gelling) metal salt and a water-insoluble metal salt seem to have less influence on the coloration or discoloration of the solid preparation in that order. Compared with the metal salt of the inorganic acid, the metal salt of the organic acid has a larger effect on the coloration or discoloration of the solid preparation; compared with the polyvalent metal salt such as the alkaline earth metal salt, the alkali metal salt seems to produce the coloration or discoloration of the solid preparation. Thus, the metal salt preferably includes a water-insoluble metal salt of an inorganic acid (particularly, a polyvalent metal salt such as an alkaline earth metal salt), a water-insoluble metal salt of an organic acid (particularly, a polyvalent metal salt such as an alkaline earth metal salt), and a water-swelling metal salt of an organic acid (particularly, a polyvalent metal salt such as an alkaline earth metal salt). Among them, the water-insoluble metal salt of the inorganic acid (particular, the polyvalent metal salt such as the alkaline earth metal salt) is particularly preferred.

Among such carriers, the carrier in the form of the metal salt of the organic acid (or a metal salt of an organic compound) easily reduces the stability of the active ingredient, different from the carrier in the form of the polyvalent

metal salt of the inorganic acid, resulting in the discoloration or coloration of the solid preparation. For these reasons, it is preferred that the proportion of the metal salt of the organic acid in the carrier be low in terms of metal. Specifically, it is preferred that the carrier have a low metal content even in a case where the carrier contains the metal salt of the organic acid; it is further preferred that the carrier (b) contain a carrier (a first carrier) that is not a metal salt-form or that is a non-metal salt-form and/or a carrier (a second carrier) that is a polyvalent metal salt-form of an inorganic acid, in other words, it is further preferred that the carrier (b) be free from a carrier (a third carrier) that is a metal salt-form of an organic acid; and it is particularly preferred that the carrier contain the carrier (the first carrier) that is not a metal salt-form or that is a non-metal salt-form.

In this description and claims, solubilities of carriers are measured in accordance with The Japanese Pharmacopoeia Eighteenth Edition, General Notice 30; when the volume of water required for dissolving 1 g of a powdered carrier is less than 30 mL, this means that the carrier is "water-soluble", and when the volume of water is not less than 100 mL, this means that the carrier is "water-insoluble".

The term "water-swelling" means a component that swells or gels by adding water. For example, such a component includes a metal salt of a cross-linked body in which an organic acid itself has a cross-linked structure (for example, a croscarmellose sodium) and a cross-linked body in which organic acids are crosslinked with a polyvalent metal salt (for example, a carmellose calcium).

The carrier (b) contains the carrier (the first carrier) that is not a metal salt-form and/or the carrier (the second carrier) that is a polyvalent metal salt-form of an inorganic acid.

[First carrier]

Examples of the first carrier, that is, the carrier that is not a metal salt-form, may include a saccharide or a sugar alcohol such as a lactose (or a lactose hydrate), a glucose, a sucrose, a malt sugar (a maltose or a maltose hydrate), a maltitol, a mannitol, a sorbitol, a xylitol, and an isomalt hydrate; a starch such as a corn starch and a potato starch; a soluble starch such as a pregelatinized starch and a partially pregelatinized starch; a polysaccharide such as a crystalline cellulose (including a microcrystalline cellulose), a carboxymethylcellulose (a carmellose, CMC), a dextrin, a gum acacia (or a gum arabic), a pectin, an agar, and an gelatin; a synthetic polymer such as a polyvinylpyrrolidone-series polymer [for example, a polyvinylpyrrolidone (PVP, povidone), a copolymer (copolyvidone) such as a vinylpyrrolidone-vinyl acetate copolymer, and a crosslinked polyvinylpyrrolidone (crospovidone)], a polyvinyl alcohol (PVA), a carboxyvinyl polymer, a poly(meth)acrylic acid-series polymer (a methacrylic acid copolymer such as Eudragit L, S, and L30D-55, and an aminoalkylmethacrylic acid copolymer such as Eudragit E and RS), a polylactic acid, and a polyethylene glycol (such as polyethylene glycol 6000); a cellulose ether such as a methylcellulose (MC), an ethylcellulose (EC), a hydroxyethylcellulose (HEC), a hydroxypropylcellulose (HPC), a low substituted hydroxypropylcellulose, and a hydroxypropylmethylcellulose (HPMC); a silicon oxide such as a light anhydrous silicic acid, silicon dioxide, and hydrated silicon dioxide; and a wax such as a yellow beeswax. These first carriers may be used alone or in combination of two or more. A preferred first carrier includes an organic carrier.

The proportion of the first carrier in the whole carrier (b) may be, for example, about not less than 50% by mass (e.g., about 55 to 99.5% by mass), preferably about not less than 60% by mass (e.g., about 65 to 99% by mass), further preferably about not less than 70% by mass (e.g., about 75 to 99% by mass), particularly about not less than 80% by mass (e.g., about 85 to 98.5% by mass).

[Second carrier]

Examples of the second carrier, that is, the carrier that is a polyvalent metal salt-form of an inorganic acid, may include a polyvalent metal salt of a silicic acid, e.g., a divalent silicate (or an alkaline earth metal salt of a silicic acid) such as a calcium silicate, a magnesium silicate, and a magnesium aluminosilicate, and a trivalent silicate such as a kaolin (or an aluminum silicate); a polyvalent metal salt of phosphoric acid, e.g., a divalent phosphate (or an alkaline earth metal salt of phosphoric acid) such as calcium monohydrogen phosphate, calcium dihydrogen phosphate, and anhydrous dibasic calcium phosphate (particularly a calcium phosphate); and a carbonate such as a magnesium carbonate. These second carriers may be used alone or in combination of two or more.

The proportion of the second carrier may be the same as the above-mentioned proportion of the first carrier [specifically, the proportion of the second carrier may be not less than 50% by mass, preferably not less than 60% by mass, further preferably not less than 70% by mass, particularly not less than 80% by mass (e.g., 85 to 98.5% by mass) in the whole carrier]; for example, the proportion of the second carrier in the whole carrier (b) may be about less than 50% by mass (e.g., about 0 to 49.5% by mass), preferably about less than 30% by mass (e.g., about 1 to 29% by mass), and further preferably about less than 20% by mass (e.g., about 3 to 19% by mass).

The ratio (weight ratio) of the first carrier relative to the second carrier may suitably be selected from 100/0 to 0/100 in terms of the former/the latter, and may for example be about 100/0 to 50/50, preferably about 95/5 to 55/45,

and further preferably about 90/10 to 60/40 (e.g., about 85/15 to 65/35) .

The total proportion of the first carrier and the second carrier in the whole carrier (b) may be, for example, about not less than 50% by mass (e.g., about 55 to 100% by mass), preferably about not less than 60% by mass (e.g., about 65 to 100% by mass), further preferably about not less than 70% by mass (e.g., about 75 to 99.5% by mass), and particularly about not less than 80% by mass (e.g., about 90 to 99% by mass).

The carrier (b) may contain a carrier (a third carrier) that is a metal salt-form of an organic acid in such a proportion that the third carrier hardly affects the coloration or discoloration of the solid preparation and the preparation stability.

[Third carrier]

Examples of the third carrier, that is, the carrier that is a metal salt-form of an organic acid, may include, a polysaccharide such as a sodium alginate; a metal salt of a carboxymethylcellulose (or a cross-linked body thereof), such as a carmellose sodium, a carmellose calcium, and a croscarmellose sodium; and a sodium carboxymethyl starch (a sodium starch glycolate) and a low substituted sodium carboxymethyl starch. These third carriers may be used alone or in combination of two or more. Even with such a metal salt of the organic acid (the third carrier), use of a polyvalent metal salt or a water-swelling metal salt (such as a carmellose calcium and a croscarmellose sodium) as the third carrier can allow the solid preparation to be prevented from discoloration or coloration.

The ratio of the third carrier relative to 100 parts by mass of the whole carrier (b) may be about 0 to 40 parts by mass, preferably about 0.01 to 35 parts by mass, and further preferably about 0.02 to 30 parts by mass. Depending on the kind of the third carrier, for example, the ratio may be about 0 to 30 parts by mass, preferably about 1 to 25 parts by mass, and further preferably about 2 to 20 parts by mass.

According to the present invention, a carrier that is an alkali metal salt-form of an inorganic acid (a fourth carrier) may be used in such a proportion that the carrier hardly affects the coloration or discoloration of the solid preparation and the preparation stability.

[Fourth carrier]

Examples of the fourth carrier, that is, the carrier that is an alkali metal salt-form of an inorganic acid, may include, an alkali metal salt of an inorganic acid such as sulfuric acid, hydrochloric acid, phosphoric acid, carbonic acid, and a silicic acid (for example, a sodium salt and a potassium salt) . Specific examples of the fourth carrier include an alkali metal salt of hydrochloric acid, such as sodium chloride; an alkali metal salt of phosphoric acid, such as sodium hydrogen phosphate and sodium dihydrogen phosphate; and an alkali metal salt of carbonic acid, such as sodium hydrogen carbonate. These fourth carriers may be used alone or in combination of two or more.

The ratio of the fourth carrier relative to 100 parts by mass of the whole carrier (b) may be about 0 to 30 parts by mass, preferably about 0.01 to 25 parts by mass, and further preferably about 0.02 to 20 parts by mass. Depending on the kind of the fourth carrier, for example, the ratio may be about 0 to 15 parts by mass, preferably about 1 to 10 parts by mass, and further preferably about 1.5 to 5 parts by mass.

According to the present invention, the carrier (b) contains the diluent (b-1), the binder (b-2), and/or the disintegrator (b-3). Among the binder (b-2) and the disintegrator (b-3), at least the disintegrator (b-3) is used. To greater or lesser degrees, since a component may serve two or more functions or roles, there are cases in which the component is included in two or more carrier component categories.

[(b-1) Diluent]

Examples of the diluent (a first diluent) (b-1) classified into the first carrier may include the following diluent components (1-1a): for example, a saccharide or a sugar alcohol such as a lactose (or a lactose hydrate), a glucose, a sucrose, a malt sugar (a maltose or a maltose hydrate), a maltitol, a mannitol, a sorbitol, a xylitol, and an isomalt hydrate; a starch such as a corn starch and a potato starch; a soluble starch such as a pregelatinized starch and a partially pregelatinized starch; a polysaccharide such as a crystalline cellulose (including a microcrystalline cellulose), a carboxymethylcellulose (a carmellose, CMC), a dextrin, and a gum acacia (or a gum arabic); a low substituted hydroxypropylcellulose; a synthetic polymer such as a polyvinylpyrrolidone-series polymer [for example, a polyvinylpyrrolidone (PVP, povidone), a copolymer (copolyvidone) such as a vinylpyrrolidone-vinyl acetate copolymer, and a crosslinked polyvinylpyrrolidone (crospovidone)]; and a silicon oxide such as a light anhydrous silicic acid, silicon dioxide, and hydrated silicon dioxide.

From the viewpoint that the solid preparation has less or no discoloration or coloration and has an excellent preparation stability, preferred examples of the first diluent among the diluent components (1-1a) may include the following diluent components (1-1b): for example, a saccharide or a sugar alcohol such as a lactose (or a lactose hydrate), a

starch such as a corn starch, a polysaccharide such as a crystalline cellulose, and a silicon oxide such as a light anhydrous silicic acid, silicon dioxide, and hydrated silicon dioxide.

Further preferred examples of the first diluent among the diluent components (1-1a) may include the following diluent components (1-1c): for example, a saccharide or a sugar alcohol such as a lactose (or a lactose hydrate), a starch such as a corn starch, and a polysaccharide such as a crystalline cellulose.

Examples of the diluent (b-1) (a second diluent) classified into the second carrier may include the following diluent components (2-1a): for example, a polyvalent metal salt of a silicic acid, e.g., a divalent silicate (or an alkaline earth metal salt of a silicic acid) such as a calcium silicate, a magnesium silicate, and a magnesium aluminosilicate, and a trivalent silicate such as a kaolin; and a polyvalent metal salt of phosphoric acid, e.g., a divalent phosphate (or an alkaline earth metal salt of phosphoric acid) such as calcium monohydrogen phosphate, calcium dihydrogen phosphate, and anhydrous dibasic calcium phosphate.

From the viewpoint that the solid preparation has less or no discoloration or coloration and has an excellent preparation stability, preferred examples of the second diluent among the diluent components (2-1a) may include the following diluent components (2-1b): for example, a polyvalent metal salt of phosphoric acid, such as calcium monohydrogen phosphate, calcium dihydrogen phosphate, and anhydrous dibasic calcium phosphate.

Further preferred examples of the second diluent among the diluent components (2-1a) may include the following diluent components (2-1c): for example, a polyvalent metal salt of phosphoric acid, such as anhydrous dibasic calcium phosphate (in particular, a calcium phosphate).

These diluents (b-1) may be used alone or in combination of two or more. As the diluent (b-1), there may preferably be used the diluent component(s) (1-1c) as the first diluent, and/or the diluent component(s) (2-1c) as the second diluent.

The ratio (weight ratio) of the first diluent and the second diluent may be selected from a range of, for example, 100/0 to 0/100 in terms of the former/the latter, and may be preferably about 100/0 to 5/95, further preferably about 95/5 to 10/90, and particularly about 90/10 to 20/80 (for example, about 85/15 to 30/70).

The total proportion of the first diluent and the second diluent in the carrier (b) may be, for example, not less than about 50% by mass (e.g., about 55 to 99.5% by mass), preferably not less than about 60% by mass (e.g., about 65 to 99% by mass), and further preferably not less than about 70% by mass (e.g., about 75 to 90% by mass).

According to the present invention, as the diluent (b-1), a diluent (a third carrier) that is a metal salt-form of an organic acid may be used in such a proportion that the third carrier hardly influences the coloration or discoloration of the solid preparation and the preparation stability. Examples of the diluent (a third diluent) (b-1) classified into such a third carrier may include the following diluent components (3-1a): for example, a polysaccharide such as a sodium alginate; a metal salt of a carboxymethylcellulose (or a cross-linked body thereof), such as a carmellose sodium, a carmellose calcium, and a croscarmellose sodium; and a sodium carboxymethyl starch (a sodium starch glycolate) and a low substituted sodium carboxymethyl starch.

Preferred examples of the third diluent among the diluent components (3-1a) may include the following diluent components (3-1b): for example, a metal salt of a carboxymethylcellulose (or a cross-linked body thereof); and a sodium carboxymethyl starch (a sodium starch glycolate).

Further preferred examples of the third diluent among the diluent components (3-1a) may include the following diluent components (3-1c): for example, a metal salt of a carboxymethylcellulose (or a cross-linked body thereof).

The proportion of the diluent (the third diluent) that is a metal salt-form of an organic acid may be about 0 to 50% by mass, preferably about 0.01 to 30% by mass, and further preferably about 0.02 to 10% by mass, relative to the total amount of the diluent (b-1); depending on the kind of the diluent, for example, the third diluent may be contained in a proportion of about 0 to 10% by mass, preferably about 0.01 to 3% by mass, and further preferably about 0.02 to 1% by mass, relative to the total amount of the diluent (b-1), and the diluent (b-1) is particularly preferably free from (or substantially free from) the third diluent.

The total amount of the diluent (in particular, including the diluent in the above-mentioned preferred embodiment, specifically, the total of the first diluent and the second diluent) per preparation (for example, per plain tablet or uncoated tablet) may be about 50 to 99.7% by mass (for example, about 60 to 99.5% by mass), preferably about 65 to 99% by mass, further preferably about 70 to 98.5% by mass, and more preferably about 75 to 98% by mass.

[(b-2) Binder]

Examples of the binder (a first binder) (b-2) classified into the first carrier may include the following binder components (1-2a): for example, a soluble starch such as a pregelatinized starch and a partially pregelatinized starch; a starch such as a corn starch and a potato starch; a polysaccharide such as a crystalline cellulose (including a microcrystalline cellulose), a carboxymethylcellulose (a carmellose, CMC), a gum acacia (or a gum arabic), a dextrin, a pectin, an agar, and an gelatin; a synthetic polymer such as a polyvinylpyrrolidone-series polymer [for example, a polyvinylpyrrolidone (PVP, povidone), a copolymer (copolyvidone) such as a vinylpyrrolidone-vinyl acetate copolymer], a polyvinyl

alcohol (PVA), a carboxyvinyl polymer, a poly(meth)acrylic acid-series polymer (a methacrylic acid copolymer such as Eudragit L, S, and L30D-55, and an aminoalkylmethacrylic acid copolymer such as Eudragit E and RS), a polylactic acid, and a polyethylene glycol (such as polyethylene glycol 6000); a cellulose ether such as a methylcellulose (MC), an ethylcellulose (EC), a hydroxyethylcellulose (HEC), a hydroxypropylcellulose (HPC), and a hydroxypropylmethylcellulose (HPMC); a saccharide or a sugar alcohol such as a lactose (or a lactose hydrate), a glucose, a sucrose, a malt sugar (a maltose or a maltose hydrate), a maltitol, a mannitol, and a sorbitol; and a wax such as a yellow beeswax.

From the viewpoint that the solid preparation has less or no discoloration or coloration and has an excellent preparation stability, preferred examples of the first binder among the binder components (1-2a) may include the following binder components (1-2b): for example, a carboxymethylcellulose (a carmellose, CMC); a synthetic polymer, e.g., a polyvinylpyrrolidone-series polymer such as a polyvinylpyrrolidone (PVP, povidone), and a poly(meth)acrylic acid-series polymer; and a cellulose ether such as an ethylcellulose (EC) and a hydroxypropylmethylcellulose (HPMC).

Further preferred examples of the first binder among the binder components (1-2a) may include the following binder components (1-2c): for example, a polyvinylpyrrolidone-series polymer such as a polyvinylpyrrolidone (PVP, povidone), and a cellulose ether such as an ethylcellulose (EC) and a hydroxypropylmethylcellulose (HPMC).

Examples of the binder (b-2) (a second binder) corresponding to the second carrier may include the following binder components (2-2a): for example, a polyvalent metal salt of a silicic acid, such as an aluminum silicate; a polyvalent metal salt of carbonic acid, such as calcium carbonate; and a polyvalent metal salt of sulfuric acid, such as aluminum sulfate.

From the viewpoint that the solid preparation has less or no discoloration or coloration and has an excellent preparation stability, preferred examples of the second binder among the binder components (2-2a) may include the following binder components (2-2b): for example, a polyvalent metal salt of a silicic acid, such as an aluminum silicate; and a polyvalent metal salt of carbonic acid, such as calcium carbonate.

These binders (b-2) may be used alone or in combination of two or more. As the binder (b-2), there may preferably be used the binder component(s) (1-2c) as the first binder, and/or the binder component(s) (2-2b) as the second binder.

According to the present invention, since the disintegrator that is a metal salt-form of an organic acid (the binder (b-2) and/or the third binder as the third carrier) has a small ratio relative to the diluent, use of the disintegrator has a small influence on the coloration or discoloration of the preparation. Thus, the binder (b-2) may contain a binder (a third binder) that is a metal salt-form of an organic acid, corresponding to the third carrier, in such a proportion that the third binder hardly affects the discoloration or coloration of the solid preparation and the preparation stability. Examples of such a third binder may include the following binder components (3-2a): for example, a polysaccharide such as a sodium alginate; a metal salt of a carboxymethylcellulose (or a cross-linked body thereof), such as a carmellose sodium, a carmellose calcium, and a croscarmellose sodium; a sodium carboxymethyl starch (a sodium starch glycolate), and a low substituted sodium carboxymethyl starch.

Preferred examples of the third binder among the binder components (3-2a) may include the following binder components (3-2b): for example, a metal salt of a carboxymethylcellulose (or a cross-linked body thereof), such as a carboxymethylcellulose sodium (a carmellose sodium); and a sodium carboxymethyl starch (a sodium starch glycolate).

Further preferred examples of the third binder among the binder components (3-2a) may include the following binder components (3-2c): for example, an alkali metal salt of a carboxymethylcellulose (or a cross-linked body thereof), such as a carboxymethylcellulose sodium (a carmellose sodium).

The proportion of the binder (the third binder) that is a metal salt-form of an organic acid may be about 0 to 100% by mass, preferably about 0.1 to 75% by mass, and further preferably about 0.2 to 50% by mass (for example, about 0.5 to 30% by mass), relative to the total amount of the binder (b-2); depending on the kind of the binder, for example, the binder (the third binder) may be contained in a proportion of about 0 to 10% by mass, preferably about 0.1 to 5% by mass, and further preferably about 0.5 to 3% by mass, relative to the total amount of the binder (b-2), and the binder (b-2) is particularly preferably free from (or substantially free from) the third binder.

The total amount of the binder (b-2) (in particular, including the binder in a preferred embodiment, specifically the total of the first binder and the second binder) relative to 100 parts by mass of the diluent (b-1) may be, for example, about 0.1 to 100 parts by mass (for example, about 0.1 to 80 parts by mass), preferably about 0.1 to 50 parts by mass, further preferably about 0.5 to 30 parts by mass, and more preferably about 1 to 15 parts by mass (for example, about 2 to 10 parts by mass); depending on the kind of the binder, for example, the total amount of the binder (b-2) relative to 100 parts by mass of the diluent (b-1) may be about 0.5 to 10 parts by mass, preferably about 1 to 5 parts by mass, and further preferably about 1.5 to 3 parts by mass.

The total amount of the binder (in particular, including the binder in a preferred embodiment) relative to 100 parts by mass of the carrier (b) may be, for example, about 0.01 to 50 parts by mass, preferably about 0.05 to 40 parts by mass, further preferably about 0.1 to 30 parts by mass, and more preferably about 0.5 to 20 parts by mass (for example, about 1 to 15 parts by mass); depending on the kind of the binder, for example, the total amount of the binder relative to 100 parts by mass of the carrier (b) may be about 0.5 to 10 parts by mass, preferably about 1 to 5 parts by

mass, and further preferably about 1.5 to 3 parts by mass.

[(b-3) Disintegrator]

The disintegrator (b-3) (a first disintegrator) as the first carrier may include the following disintegrator components (1-3a); for example, a soluble starch such as a pregelatinized starch and a partially pregelatinized starch; a starch such as a corn starch and a potato starch; a polysaccharide such as a carboxymethylcellulose (a carmellose, CMC) and a low substituted carboxymethylcellulose; a synthetic polymer such as a polyvinylpyrrolidone-series polymer [for example, a crosslinked polyvinylpyrrolidone (crospovidone)]; a low substituted hydroxypropylcellulose; and a saccharide or a sugar alcohol such as a lactose (or a lactose hydrate), a glucose, a sucrose, a malt sugar (a maltose or a maltose hydrate), a maltitol, a mannitol, and a sorbitol.

From the viewpoint that the solid preparation has less or no discoloration or coloration and has an excellent preparation stability, preferred examples of the first disintegrator among the disintegrator components (1-3a) may include the following disintegrator components (1-3b): for example, a soluble starch such as a pregelatinized starch and a partially pregelatinized starch; a polysaccharide such as a low substituted carboxymethylcellulose; a polyvinylpyrrolidone-series polymer such as a crospovidone; and a low substituted hydroxypropylcellulose.

Further preferred examples of the first disintegrator among the disintegrator components (1-3a) may include the following disintegrator components (1-3c): for example, a soluble starch such as a pregelatinized starch; a polysaccharide such as a low substituted carboxymethylcellulose; a polyvinylpyrrolidone-series polymer such as a crospovidone; and a low substituted hydroxypropylcellulose.

The disintegrator (b-3) (a second disintegrator) as the second carrier may include the following disintegrator components (2-3a): for example, a polyvalent metal salt of a silicic acid, e.g., a divalent silicate (or an alkaline earth metal salt of a silicic acid) such as a calcium silicate, a magnesium silicate, and a magnesium aluminosilicate, and a trivalent silicate such as a kaolin (or an aluminum silicate); a polyvalent metal salt of phosphoric acid, e.g., a divalent phosphate (or an alkaline earth metal salt of phosphoric acid) such as calcium monohydrogen phosphate, calcium dihydrogen phosphate, and anhydrous dibasic calcium phosphate; and a carbonate such as a magnesium carbonate.

From the viewpoint that the solid preparation has less or no discoloration or coloration and has an excellent preparation stability, preferred examples of the second disintegrator among the disintegrator components (2-3a) may include the following disintegrator components (2-3b): for example, a polyvalent metal salt of a silicic acid, such as an aluminum silicate; a polyvalent metal salt of phosphoric acid, such as calcium dihydrogen phosphate; and a polyvalent metal salt of carbonic acid, such as a magnesium carbonate.

Further preferred examples of the second disintegrator among disintegrator components (2-3a) may include the following disintegrator components (2-3c): for example, a polyvalent metal salt of a silicic acid, such as an aluminum silicate; and a polyvalent metal salt of phosphoric acid, such as calcium dihydrogen phosphate.

These disintegrators (b-3) may be used alone or in combination of two or more. As the disintegrator (b-3) in the present invention, there may preferably be used the disintegrator component(s) (1-3c) as the first disintegrator and/or the disintegrator component(s) (2-3c) as the second disintegrator.

According to the present invention, since the disintegrator has a small ratio relative to the diluent, use of the disintegrator that is a metal salt-form of an organic acid (the disintegrator (b-3) classified into the third carrier, or the third disintegrator) has a small effect on the coloration or discoloration of the preparation. Thus, the third disintegrator (b-3) may be contained or used in such a proportion that the third disintegrator hardly influences the discoloration or coloration of the solid preparation and the preparation stability. Examples of such a third disintegrator (3-3a) may include the components exemplified as the third diluent, for example, (3-3a) disintegrator components: a polysaccharide such as a sodium alginate; a metal salt of a carboxymethylcellulose (or a cross-linked body thereof), such as a carmellose sodium, a carmellose calcium, and a croscarmellose sodium; and a sodium carboxymethyl starch (a sodium starch glycolate), and a low substituted sodium carboxymethyl starch.

Preferred examples of the third disintegrator among the disintegrator components (3-3a) may include the following disintegrator components (3-3b): for example, a metal salt of a carboxymethylcellulose (or a cross-linked body thereof), such as a carmellose sodium, a carmellose calcium, and a croscarmellose sodium; and a sodium carboxymethyl starch (a sodium starch glycolate), and a low substituted sodium carboxymethyl starch.

Further preferred examples of the third disintegrator among the disintegrator components (3-3a) may include the following disintegrator components (3-3c): for example, a metal salt of a carboxymethylcellulose (or a cross-linked body thereof), such as a carmellose calcium and a croscarmellose sodium; and a sodium carboxymethyl starch (a sodium starch glycolate).

The proportion of the disintegrator (the third disintegrator) in a metal salt-form of an organic acid may be about 0 to 100% by mass, preferably about 0.01 to 30% by mass, and further preferably about 0.02 to 10% by mass (for example, about 0.05 to 8% by mass), relative to total amount of the disintegrator (b-3); depending on the kind of the disintegrator (b-3), for example, the disintegrator (the third disintegrator) may be contained in a proportion of about 0 to

10% by mass, preferably about 0.01 to 3% by mass, and further preferably about 0.02 to 1% by mass, relative to the total amount of the disintegrator (b-3), and the disintegrator (b-3) is particularly preferably free from (or substantially free from) the third disintegrator.

The total amount of the disintegrator (b-3) (in particular, including the disintegrator in a preferred embodiment) relative to 100 parts by mass of the diluent (b-1) may be, for example, about 0.1 to 100 parts by mass, preferably about 0.1 to 50 parts by mass, further preferably about 0.5 to 30 parts by mass, and more preferably about 1 to 25 parts by mass (for example, about 1.5 to 20 parts by mass); the total amount of the disintegrator (b-3) relative to 100 parts by mass of the diluent (b-1) may be, for example, 0.5 to 10 parts by mass, preferably 0.75 to 5 parts by mass, further preferably 0.1 to 4 parts by mass, and particularly 0.5 to 3 parts by mass.

The total amount of the disintegrator (b-3) (in particular, including the disintegrator in a preferred embodiment, specifically the total of the first disintegrator and the second disintegrator) relative to 100 parts by mass of the carrier (b) may be, for example, about 0.01 to 50 parts by mass, preferably about 0.05 to 40 parts by mass, further preferably about 0.1 to 30 parts by mass, and more preferably about 0.5 to 20 parts by mass (for example, about 1 to 15 parts by mass); the total amount of the disintegrator (b-3) relative to 100 parts by mass of the carrier (b) may be, for example, 0.5 to 10 parts by mass, preferably 0.75 to 7.5 parts by mass, further preferably 0.1 to 6 parts by mass, and particularly 1 to 5 parts by mass (for example, 1 to 3 parts by mass).

These carriers (b) may be used alone or in combination of two or more. As these carriers, use of the carrier (the third carrier) in a metal salt-form of an organic acid or the alkali metal salt of an inorganic acid (the fourth carrier) in excess of a predetermined ratio may cause the coloration or discoloration of the preparation. According to the present invention, however, the carrier (the third carrier) in a metal salt-form of an organic acid and/or the alkali metal salt of the inorganic acid (the fourth carrier) may be used in combination with the carrier (the first carrier) in a non-metal salt-form and/or the carrier (the second carrier) in a polyvalent metal salt-form of an inorganic acid unless the combination use reduces the stability of the active ingredient or causes significant discoloration or coloration of the solid preparation.

[Metal content]

The carrier containing a carrier that is not a metal salt-form of an organic acid allows stably less or no discoloration and/or coloration of the solid preparation over a long period of time. According to the present invention, the carrier (b) is the carrier that is not a metal salt-form of an organic acid (the first carrier, specifically, a carrier free from or substantially free from a carrier that is a metal salt-form of an organic acid) or the carrier that is a polyvalent metal salt-form of an inorganic acid (the second carrier), and thus the solid preparation of the present invention has an excellent stability without discoloration or coloration even when stored over a long period of time. Moreover, even when the solid preparation contains the carrier in a metal salt-form of an organic acid (the third carrier) or the carrier in an alkali metal salt-form of an inorganic acid (the fourth carrier), the discoloration or coloration of the solid preparation can effectively be prevented in a small predetermined proportion of the metal content of the metal salt of the organic acid.

As the metal content of the metal salt of the organic acid (the third carrier) in the carrier (b) increases, the discoloration or coloration of the solid preparation according to the present invention seems to increase. Moreover, since the alkali metal salt of the inorganic acid (or the alkali metal halide, the fourth carrier) is also water-soluble, it is predicted that an increase in the metal content produces an increased discoloration or coloration of the solid preparation according to the present invention.

For the effect of the metal content of the metal salt of the organic acid in the carrier (b) on the discoloration or coloration of the solid preparation, a croscarmellose sodium having a metal content of 9.50% by mass and a carmellose calcium having a metal content of 5.74% by mass were used to examine the discoloration or coloration of the solid preparation after 4 weeks and 6 weeks of storage under the conditions of a temperature of 40°C and a relative humidity of 75%. The results showed that, when the content of the metal salt of the organic acid in the carrier (b) was not more than 10% by mass (for example, 0 to 10% by mass) in terms of metal, the solid preparation had a small color difference ($\Delta E^*$) both after 4 weeks and 6 weeks of storage and the discoloration or coloration of the solid preparation was not seen over a long period of term. Thus, the carrier (b) may contain the carrier that is the metal salt-form of the organic acid in a proportion of not more than 10% by mass (for example, 0 to 10% by mass), and particularly not more than 5% by mass (for example, 0 to 3% by mass), in terms of metal.

Specifically, for the carrier (b) containing the third carrier and the fourth carrier, the proportion of the third carrier and the fourth carrier may be, in terms of metal, about not more than 10% by mass (about 0 to 10% by mass, for example, about 0 to 9% by mass), preferably about not more than 8% by mass (for example, about 0.001 to 7.5% by mass), further preferably about not more than 7% by mass (for example, about 0.01 to 6% by mass), more preferably about not more than 5% by mass (for example, about 0.02 to 5% by mass), and particularly about not more than 4.5% by mass (for example, about 0.05 to 3% by mass); the proportion may be not more than 4% by mass (for example, 0 to 3.5% by mass), and particularly not more than 3% by mass (for example, 0 to 2.5% by mass). In this description and claims, a metal content of a carrier containing a component with an identifiable structure was calculated on the basis of the metal

content of the component and the ratio of the component relative to the whole carrier, and a metal content of a carrier containing a component with an unidentifiable structure was calculated by atomic absorption spectrometry.

The binder and the disintegrator are practically used in a small ratio relative to the diluent, and thus have a small effect on the coloration or discoloration of the preparation even in a case where the binder and the disintegrator have a metal salt-form. The metal content of the binder (in particular, including the binder in a preferred embodiment), particularly the metal content of the metal salt of the organic acid, relative to the whole carrier (b) may be, in terms of metal, about not more than 5% by mass (for example, about 0.001 to 5% by mass), preferably not more than 4% by mass (for example, about 0.01 to 3.5% by mass), and further preferably not more than 3% by mass (for example, about 0.05 to 2.5% by mass). The metal content of the disintegrator (in particular, including the disintegrator in a preferred embodiment), particularly the metal content of the metal salt of the organic acid, relative to the whole carrier (b) may be, in terms of metal, about not more than 10% by mass (for example, about 0.001 to 9% by mass), preferably about not more than 8% by mass (for example, about 0.01 to 7.5% by mass), further preferably about not more than 5% by mass (for example, about 0.05 to 5% by mass), and more preferably about not more than 4.5% by mass (for example, about 0.1 to 3% by mass).

The solid preparation (or the urination disorder-improving agent) according to the present invention may further contain a lubricant.

[Lubricant]

The lubricant may be, for example, a water-soluble lubricant [for example, sodium lauryl sulfate; and a water-soluble synthetic polymer such as a polyethylene glycol (e.g., polyethylene glycol 6000)]. From the viewpoint that the solid preparation retains the stability of the active ingredient and has less or no discoloration or coloration, the lubricant may be a water-insoluble lubricant [for example, a higher unsaturated fatty acid or a metal salt thereof, such as sodium stearyl fumarate; a higher saturated fatty acid or a metal salt (a polyvalent metal salt) thereof, such as stearic acid, magnesium stearate, aluminum stearate, and calcium stearate; an ester of a higher fatty acid, such as a glycerol ester of a fatty acid, a sucrose ester of a fatty acid, glyceryl monostearate, and a hydrogenated oil; a wax such as a carnauba wax, a yellow beeswax, and a Japan wax; and a synthetic polymer, e.g., a paraffin such as a liquid paraffin]; and a mineral such as a talc. These lubricants may be used alone or in combination of two or more. Preferred examples of the lubricant include a water-insoluble lubricant, particularly a higher saturated fatty acid or a metal salt (a polyvalent metal salt) thereof, such as stearic acid or magnesium stearate; and a mineral such as a talc. Even use of the metal salt such as magnesium stearate as the lubricant does not seem to adversely affect the stability of the active ingredient, or the discoloration or coloration of the solid preparation, probably because such a metal salt is water-insoluble and highly hydrophobic.

Since the lubricant has a small ratio relative to the diluent (b-1), even use of the lubricant having a metal salt-form has a small effect on the coloration or discoloration of the preparation.

The total amount of the lubricant relative to 100 parts by mass of the diluent (b-1) may be, for example, about 0.01 to 20 parts by mass, preferably about 0.1 to 10 parts by mass, further preferably about 0.3 to 7 parts by mass, and more preferably about 0.5 to 5 parts by mass.

The total amount of the lubricant relative to 100 parts by mass of the carrier (b) may be, for example, about 0.01 to 20 parts by mass, preferably about 0.05 to 10 parts by mass, and further preferably about 0.1 to 5 parts by mass.

As the coating agent, there may be used, for example, a saccharide or a sugar, a cellulose derivative such as an ethylcellulose and a hydroxymethylcellulose, a polyoxyethylene glycol, a cellulose acetate phthalate, a hydroxypropylmethylcellulose phthalate, a methyl methacrylate-(meth)acrylic acid copolymer, and Eudragit (a copolymer of methacrylic acid and acrylic acid). Such a coating agent may be an enteric component (e.g., a cellulose phthalate, a hydroxypropylmethylcellulose phthalate, and a methyl methacrylate-(meth)acrylic acid copolymer) or a gastric soluble component comprising a polymer (e.g., Eudragit) containing a basic component such as a dialkylaminoalkyl(meth)acrylate. The preparation may be a capsule having such an enteric component or gastric soluble component as a capsule shell.

In the solid preparation, a known additive can suitably be used depending on an administration route, a dosage form, or others. Such an additive may include, for example, a disintegrant aid, an antioxidation agent or an antioxidant, a stabilizer, an antiseptic agent or a preservative, a fungicide or antibacterial agent, an antistatic agent, a corrigent or a masking agent, a coloring agent, a deodorant or a perfume, an algefacient, and an antifoaming agent. These additives may be used alone or in combination of two or more.

In the pharmaceutical composition or physiologically active composition in combination with the carrier (b) (e.g., a pharmacologically or physiologically acceptable carrier), the amount of the 2-oxapregnane compound represented by the formula (1) or the pharmaceutically acceptable salt thereof as the active ingredient (a) may suitably be selected according to the species, age, body weight, and condition (e.g., a performance status, a condition of a disease, a presence of a complication) of the subject to be administered, the duration (or period or schedule) of administration, the dosage form, the administration method (or route), and others. For example, the amount of the 2-oxapregnane compound or

the pharmaceutically acceptable salt thereof per unit preparation (for example, per tablet) may be about 0.1 to 20% by mass, preferably about 0.5 to 15% by mass, and further preferably about 1 to 10% by mass.

The urination disorder-improving agent (the pharmaceutical composition or preparation) according to the present invention may be prepared using the active ingredient and, in addition, a carrier component and an optional additive or other components, with a conventional preparation manner (for example, a production process described in The Japanese Pharmacopoeia Eighteenth Edition or a process in accordance with the production process).

The 2-oxapregnane compound or the pharmaceutically acceptable salt thereof, which is a known compound as described above, has been used over the years and has a high stability and a high safety.

The urination disorder-improving agent (the pharmaceutical composition or preparation) according to the present invention can safely be administered to human beings and non-humans, usually mammals (for example, human beings, mice, rats, rabbits, dogs, cats, bovines, horses, pigs, and monkeys). The amount to be administered (dosage or dose) may be selected according to the species, age, body weight, and condition (e.g., a performance status, a condition of a disease, a presence of a complication) of the subject to be administered, the duration (or period or schedule) of administration, the dosage form, the administration method (or route), and others. For example, the dosage for human beings (daily dose) may be, in terms of the compound represented by the formula (1), about 0.01 to 100 mg/day, preferably about 0.05 to 75 mg/day (e.g., about 0.1 to 50 mg/day), and particularly about 0.1 to 30 mg/day (e.g., about 0.5 to 25 mg/day, particularly about 1 to 20 mg/day).

The administration method (or route) may be an oral administration or a local or parenteral administration (for example, transrectal administration and transvaginal administration). In a preferred embodiment, the urination disorder-improving agent (the pharmaceutical composition or preparation) according to the present invention is orally administered. The frequency of administration is not particularly limited to a specific one, and may, for example, be once a day or if necessary a plurality times a day (e.g., twice to three times a day) .

As described above, the present invention also includes a method for treating, or improving or alleviating (or mitigating) the urination disorder (and a method for improving or promoting urination), the method including administering the 2-oxapregnane compound or the pharmaceutically acceptable salt thereof as an active ingredient to a subject with the urination disorder or administering the urination disorder-improving agent (the pharmaceutical composition or preparation) to a subject with the urination disorder. Further, the present invention includes use of the 2-oxapregnane compound or the pharmaceutically acceptable salt thereof as an active ingredient for treating, or improving or alleviating (or mitigating) the urination disorder (and improving or promoting urination), or use of the urination disorder-improving agent (the pharmaceutical composition or preparation) for treating, or improving or alleviating (or mitigating) the urination disorder (and improving or promoting urination). The present invention also includes use of the 2-oxapregnane compound or the pharmaceutically acceptable salt thereof for producing the urination disorder-improving agent, the urination improver, or the urination promotor.

(A) Preferred embodiments of the present invention are as follows.

(A-1) The proportion of a carrier that is not a metal salt-form (a first carrier) and/or a carrier that is a polyvalent metal salt-form of an inorganic acid (a second carrier) in a whole carrier (b) is not less than 50% by mass (for example, 55 to 99.5% by mass), preferably not less than 60% by mass (for example, 65 to 99% by mass), further preferably not less than 70% by mass (for example, 75 to 99% by mass), and particularly not less than 80% by mass (for example, 85 to 98.5% by mass).

(A-2) In the embodiment (A-1), the first carrier contains at least an organic carrier.

(A-3) In the embodiment (A-1) or (A-2), the first carrier contains at least one member selected from

a saccharide or a sugar alcohol such as a lactose (or a lactose hydrate),
a starch such as a corn starch,
a soluble starch such as a pregelatinized starch,
a polysaccharide such as a crystalline cellulose and a carboxymethylcellulose (a carmellose, CMC),
a synthetic polymer such as a polyvinylpyrrolidone-series polymer [e.g., a polyvinylpyrrolidone (PVP, povidone), a copolymer (copolyvidone) such as a vinylpyrrolidone-vinyl acetate copolymer, and a crosslinked polyvinylpyrrolidone (crospovidone)], a polyvinyl alcohol (PVA), a carboxyvinyl polymer, a poly(meth)acrylic acid-series polymer, a polylactic acid, and a polyethylene glycol,
a cellulose ether such as an ethylcellulose (EC), a low substituted hydroxypropylcellulose, and a hydroxypropylmethylcellulose (HPMC),
a silicon oxide such as a light anhydrous silicic acid, silicon dioxide, and hydrated silicon dioxide, and
a wax such as a yellow beeswax.

(A-4) In the embodiment (A-1) or (A-2), the first carrier contains at least one member selected from

a saccharide or a sugar alcohol such as a lactose (or a lactose hydrate),
a starch such as a corn starch,
a soluble starch such as a pregelatinized starch,
a polysaccharide such as a crystalline cellulose, a polyvinylpyrrolidone-series polymer such as a povidone and a crospovidone, and
a silicon oxide such as a light anhydrous silicic acid, silicon dioxide, and hydrated silicon dioxide.

(A-5) In the embodiment (A-1) or (A-2), the first carrier contains at least one member selected from

a saccharide or a sugar alcohol such as a lactose (or a lactose hydrate),
a starch such as a corn starch,
a soluble starch such as a pregelatinized starch,
a polysaccharide such as a crystalline cellulose, and
a polyvinylpyrrolidone-series polymer such as a povidone and a crospovidone.

(A-6) In any one of the embodiments (A-1) to (A-5), the second carrier contains an inorganic carrier which is an alkaline earth metal salt of at least one inorganic acid selected from sulfuric acid, phosphoric acid, carbonic acid, and silicic acid.
(A-7) In any one of the embodiments (A-1) to (A-6), the ratio (weight ratio) of the first carrier and the second carrier is 100/0 to 0/100 or 100/0 to 50/50, preferably 95/5 to 50/50, further preferably 90/10 to 55/45, and more preferably 85/15 to 60/40 in terms of the former/the latter.
(A-8) In any one of the embodiments (A-1) to (A-7), the carrier (b) is the first carrier.
(A-9) In any one of the embodiments (A-1) to (A-8), the carrier (b) contains a diluent, and a binder and/or a disintegrator, the ratio of the binder relative to 100 parts by mass of the diluent is 0 to 15 parts by mass, and the ratio of the disintegrator relative to 100 parts by mass of the diluent is 1 to 25 parts by mass.

(B) Further preferred embodiments of the present invention are as follows.

(B-1) A carrier contains a diluent, a binder and/or a disintegrator, and at least the diluent contains a diluent that is not a metal salt-form (a first diluent corresponding to the first carrier) and/or a diluent that is a polyvalent metal salt of an inorganic acid (a second diluent corresponding to the second carrier).
(B-2) In the embodiment (B-1), the diluent that is not a metal salt-form (the first diluent) contains at least one member selected from a saccharide or a sugar alcohol, a starch such as a corn starch, a polysaccharide such as a crystalline cellulose, a silicon oxide such as a light anhydrous silicic acid, silicon dioxide, and hydrated silicon dioxide.
(B-3) In the embodiment (B-1), the first diluent contains at least one member selected from a saccharide or a sugar alcohol, a starch such as a corn starch, and a polysaccharide such as a crystalline cellulose.
(B-4) In any one of the embodiments (B-1) to (B-3), the diluent that is the polyvalent metal salt of the inorganic acid (the second diluent) contains at least one inorganic diluent selected from alkaline earth metal salts of at least one inorganic acid selected from sulfuric acid, phosphoric acid, carbonic acid, and silicic acid.
(B-5) In any one of the embodiments (B-1) to (B-4), the carrier contains the diluent and at least the disintegrator of the binder and the disintegrator, and at least the diluent contains a diluent that is not a metal salt-form (the first diluent corresponding to the first carrier), specifically the diluent is free from a diluent that is a metal salt-form.
(B-6) In any one of the embodiments (B-1) to (B-5), the binder and/or the disintegrator is free from a binder and/or a disintegrator that is a metal salt-form of an organic acid (a third binder and/or a third disintegrator) or contains the third binder and/or the third disintegrator.
(B-7) In any one of the embodiments (B-1) to (B-6), the carrier contains the diluent that is not a metal salt-form of an organic acid (the first diluent corresponding to the first carrier), and the binder and/or the disintegrator, and the binder and/or the disintegrator is free from the binder and/or the disintegrator that is a metal salt-form of an organic acid (the third binder and/or the third disintegrator) .

(C) More preferred embodiments of the present invention are as follows.

(C-1) In a solid preparation at least containing (a) an active ingredient and (b) a carrier, a diluent as the carrier contains at least one component selected from the diluent components (1-1c) as the first diluent and the diluent components (2-1c) as the second diluent.
(C-2) In the embodiment (C-1), the carrier contains a binder and/or a disintegrator, the binder is at least one component selected from the binder components (1-2c) as the first binder, the disintegrator contains at least

one component selected from the disintegrator components (1-3c) as the first disintegrator, the disintegrator components (2-3c) as the second disintegrator, and the disintegrator components (3-3c) as the third disintegrator.
(C-3) In the embodiment (C-1), a disintegrator contains at least one component selected from the disintegrator components (1-3c) as the first disintegrator and the disintegrator components (2-3c) as the second disintegrator.
(C-4) In any one of the embodiments (C-1) to (C-3), relative to 100 parts by mass of the diluent [in particular, including the diluent components (1-1b) and (2-1b) in a preferred embodiment, especially the diluent components (1-1c) and (2-1c)], the ratio of the binder (in particular, including the binder components (1-2c) in a preferred embodiment) is 0 to 15 parts by mass and the ratio of the disintegrator (in particular, including the disintegrator components (1-3c) and/or (3-3c) in a preferred embodiment) is 1 to 25 parts by mass.

(D) Specifically preferred embodiments of the present invention are as follows.

(D-1) In a solid preparation at least containing (a) an active ingredient and (b) a carrier, the carrier contains a diluent and, of a binder and/or a disintegrator, at least the disintegrator, the diluent is at least one member selected from a lactose, a corn starch, a crystalline cellulose, and a calcium phosphate, the binder is at least one member selected from a polyvinylpyrrolidone (PVP, povidone) and an ethylcellulose, and the disintegrator is at least one member selected from a pregelatinized starch, a crospovidone, and a low substituted hydroxypropylcellulose.
(D-2) In the embodiment (D-1), relative to 100 parts by mass of the diluent (in particular, including the diluent in a preferred embodiment), the ratio of the binder (in particular, including the binder in a preferred embodiment) is 0 to 10 parts by mass and the ratio of the disintegrator is 1 to 20 parts by mass.

(E) Particularly preferred embodiments of the present invention are as follows.

(E-1) In the embodiments (A) to (D), the proportion of the metal salt in the carrier is, in terms of metal, not more than 10% by mass (for example, 0 to 9% by mass), preferably not more than 8% by mass (for example, 0 to 7% by mass), further preferably not more than 5% by mass (for example, 0 to 4.5% by mass), more preferably not more than 4% by mass (for example, 0 to 3.5% by mass), and particularly not more than 3% by mass (for example, 0 to 2.5% by mass).
(E-2) In the embodiment (E-1), the proportion of the metal salt of the organic acid (the third carrier) in the carrier is, in terms of metal, not more than 10% by mass (for example, 0 to 9% by mass), preferably not more than 8% by mass (for example, 0 to 7% by mass), further preferably not more than 5% by mass (for example, 0 to 4.5% by mass), more preferably not more than 4% by mass (for example, 0 to 3.5% by mass), and particularly not more than 3% by mass (for example, 0 to 2.5% by mass).
(E-3) In the embodiment (E-1) or (E-2), the metal salt is an alkali metal salt and/or an alkaline earth metal salt.
(E-4) In any one of the embodiments (E-1) to (E-3), the metal salt is a sodium salt and/or a calcium salt.
(E-5) In any one of the embodiments (E-1) to (E-4), the carrier is free from an alkali metal salt of an inorganic acid and an alkali metal halide (the fourth carrier).

The solid preparation according to the present invention may be specified in a combination of the matters described in this description with these embodiments (A) to (E).

EXAMPLES

The following examples are intended to describe this invention in further detail and should by no means be interpreted as defining the scope of the invention.

Test Example 1

A lower urinary tract obstruction model of rats was used as a test system. A test substance was orally administered to the lower urinary tract obstruction model repeatedly over two (2) weeks to examine the action on the urination function in the lower urinary tract obstruction model.
More specifically, the rats (female, Crl:CD(SD), 8-week-old; Charles River Laboratories Japan, Inc.) were operated to form lower urinary tract obstruction and repeatedly received oral administration of the test substance at a frequency of once a day over two (2) weeks. After the completion of the administration, each rat was subjected to bladder catheterization under anesthesia, cystometry under no anesthesia, then cystometry under urethane anesthesia, and euthanasia under deep anesthesia, and the tissues (bladder, kidney) of the rat were extracted or surgically removed. As a normal group, the rats without operation for forming lower urinary tract obstruction were used.

The test system, the test substances, and the dosage are as follows.

Normal group: 0.5 v/v% Polysorbate 80 solution, 10 mL/kg (n = 6)
Solvent group (control group): 0.5 v/v% Polysorbate 80 solution, 10 mL/kg (n = 15)
Group A: Compound A, 10 mg/kg (n = 15)
Group B3: Compound B, 3 mg/kg (n = 7)
Group B10: Compound B, 10 mg/kg (n = 7)

The names of the compound A and the compound B are as follows.

Compound A: 17α-acetoxy-6-chloro-15β-hydroxy-2-oxa-4,6-pregnadiene-3,20-dione
Compound B: TZP-4238 (17α-acetoxy-6-chloro-2-oxa-4,6-pregnadiene-3,20-dione

The residual urine volume, the urination efficiency, and the bladder capacity were measured as follows.

[Residual urine volume]

After urination, the residual urine was aspirated from the outlet end of the catheter placed in the bladder to measure the volume (mL) of the residual urine.

[Urination efficiency]

After urination, the weight of the voided urine was measured to determine the voided volume (g = mL), and then the urination efficiency was calculated by the following equation:

$$\text{Urination efficiency (\%)} = [(\text{Voided volume (mL)})/(\text{Voided volume (mL)} + \text{Residual urine volume (mL)})] \times 100$$

[Bladder capacity]

The bladder capacity was calculated from the voided volume and the residual urine volume by the following equation:

$$\text{Bladder capacity (mL)} = \text{Voided volume (mL)} + \text{Residual urine volume (mL)}$$

Moreover, the wet weight of the extracted bladder and that of the extracted kidney were measured.

Fig. 1 to Fig. 6 show the results of the residual urine volume, the urination efficiency, the bladder capacity, the wet weights of the extracted tissues (bladder, kidney), and the survival rate in the female rat lower urinary tract obstruction model. Fig. 1 shows a relationship between the residual urine volume and the test substances. Fig. 2 shows a relationship between the urination efficiency and the test substances. Fig. 3 shows a relationship between the bladder capacity and the test substances. Fig. 4 and Fig. 5 show a relationship between the bladder wet weight and the test substances and a relationship between the kidney wet weight and the test substances, respectively. Fig. 6 shows a relationship between the survival rate and the test substances.

As apparent from Fig. 1 to Fig. 3, Groups B (Group B3 and Group B10) showed significantly increased residual urine volume and bladder capacity and a reduced urination efficiency in comparison with the control group (the solvent group). In contrast, Group A showed significantly decreased residual urine volume and bladder capacity and an increased urination efficiency in comparison with the control group. Moreover, as apparent from Fig. 4 and Fig. 5, Group A was in the direction of significantly inhibiting the increase in the wet weights of the bladder and the kidney associated with lower urinary tract obstruction, in contrast, Groups B were rather in the direction of increasing these wet weights. Thus, Groups B were in the direction of not improving or the direction of rather deteriorating the urination function, in contrast, Group A showed a significant improvement of the urination function lowered due to lower urinary tract obstruction. Further,

Group A showed a significant improved urination function in the female rat, having no prostate, of the lower urinary tract obstruction model system. These results show that the compound A improves the urination disorder regardless of the presence of prostate enlargement.

Furthermore, as apparent from Fig. 6, Groups B showed a decreased survival rate of the lower urinary tract obstruction rats, in contrast, Group A showed no decrease in the survival rate. The results have confirmed that the compound A has a high safety.

It is not clear why the above-mentioned results were obtained in the female rats in spite of the fact that these test substances belong to the same 2-oxapregnane compound (progesterones). A possible reason may be as follows.

In clinical practice, it is considered that progesterone, which increases during pregnancy, relaxes the organs around the uterus including the bladder. It is reported that, with respect to rats, pregnancy and progesterone affect bladder muscarinic receptors to increase the bladder capacity or inhibit the bladder contraction (Pharmacology. 50 (3): 192-200. 1995). In addition to relaxation by progesterone, the bladder is functionally hypotonic due to mechanical compression from the rear by the pregnant uterus. Thus, progesterone may possibly induce the development or phenomenon of the residual urine or vesicoureteral reflux.

Groups B show a strong progesterone-like action and are considered or expected to inhibit the bladder contraction by the above-mentioned mechanism. In a pathological model in which the bladder function is strongly impaired, such as the female lower urinary tract obstruction model, it is possible that, for Groups B, excessive inhibition of the bladder contraction by the compound B resulted in exacerbation or deterioration of the pathology.

On the other hand, the compound A has a slight antiprogesterone action. Female rats have an estrus cycle of about four (4) days. It is possible that for Group A, the compound A antagonized endogenetic progesterone increasing according to the estrus cycle to protectively act on further inhibition of the bladder function in the lower urinary tract obstruction model.

Test Example 2

A lower urinary tract obstruction model of rats was used as a test system. A test substance was orally administered to the lower urinary tract obstruction model repeatedly over 14 days to examine the action on the urination function in the lower urinary tract obstruction model. The lower urinary tract obstruction model is not a benign prostatic hyperplasia model with enlarged prostate.

More specifically, the rats (male, Crl:CD(SD), 8-week-old; Charles River Laboratories Japan, Inc.) were operated to form lower urinary tract obstruction and repeatedly received oral administration of the test substance at a frequency of once a day over 14 days. After the completion of the administration, each rat was subjected to bladder catheterization under anesthesia and cystometry under no anesthesia and was evaluated for the residual urine volume and the urination efficiency.

The test substances and the dosage in the test system (male rats with lower urinary tract obstruction) are as follows.

Solvent group (control group): 0.5 v/v% Polysorbate 80 solution, 10 mL/kg (n = 8)
Group A: Compound A, 10 mg/kg (n = 12)
Group C: Compound C (dutasteride), 0.5 mg/kg (n = 11)

Dutasteride is a 5$\alpha$ reductase inhibitor.

The results are shown in Fig. 7 and Fig. 8.

Fig. 7 and Fig. 8 indicate that, in the male rat lower urinary tract obstruction model, Group A has a strong improvement action on the urination function (the residual urine volume, the urination efficiency) in comparison with Group C (dutasteride).

Test Example 3

High-week-old male rats (22-week-old; Charles River Laboratories Japan, Inc.) were examined and evaluated for the relationship between the test substances and the bladder and urethra contraction reaction. Since the rats are high-week-old, the rats are a model in which the prostate is predicted to be enlarged.

A test substance was orally administered to the high-week-old male rats repeatedly at a frequency of once a day over two (2) weeks, and then, from each rat, a bladder specimen was extracted. The bladder specimen was suspended at a resting tension of about 9.8 mN in a constant-temperature Magnus tube (37°C) filled with a physiological salt solution aerated with 95% $O_2$-5% $CO_2$ gas and was subjected to an isometric contraction test by applying a square-wave electrical field stimulation (EFS, Amplitude; 0.3 ms, Frequency; 1, 2, 5, 10, 20 Hz, Duration; 5s); or Carbachol (CCH, 100 nmol/L to 100 $\mu$mol/L) and KCl (40, 80 mmol/L).

The test substances and the dosage in the test system (high-week-old male rats) are as follows.

Solvent group (control group): 0.5 v/v% Polysorbate 80 solution, 10 mL/kg (n = 6)
Group A: Compound A, 10 mg/kg (n = 6)
Group C: Compound C (dutasteride), 0.3 mg/kg (n = 6)

The results are shown in Fig. 9 and Fig. 10.

Fig. 9 and Fig. 10 have revealed that the bladder contraction tension caused by square-wave electrical field stimulation (bladder efferent nerve stimulation) or Carbachol (muscarinic receptor stimulation) and KCl (depolarization-dependent $Ca^{2+}$ stimulation) is significantly strong or has a strong directionality in Group A compared with Group C.

Test Example 4

As described below, as a urination disorder model, a urination disorder model associated with diabetes was prepared to evaluate the urination disorder. This model has no benign prostatic hyperplasia and is a model of a urination disorder associated with neuropathy and smooth muscle contraction disorder.

The body weights of rats (male, Crl:CD(SD), 5-week-old; Charles River Laboratories Japan, Inc.) were measured. After the rats were fasted overnight, the blood was collected from the caudal vein to measure the fasting blood glucose. On the basis of the body weight and the fasting blood glucose level, the rats were grouped or divided into two (2) groups by randomized, multivariable block allocation. The day after the rats were grouped, a first group received intraperitoneal administration of a citrate buffer (pH 4.5) (10 mL/kg) having a concentration of 0.1 mmol/L, and a second group received intraperitoneal administration of streptozocin (STZ; Sigma-Aldrich) (50 mg/kg). Streptozocin (STZ) was administered in the form of a solution of streptozocin having a concentration of 5 mg/mL in the citrate buffer.

About one (1) week after streptozocin (STZ) was administered, the body weight and the fasting blood glucose were measured. In the streptozocin (STZ) administration group (the second group), individuals having a fasting blood glucose level of not less than 300 mg/dL were used as animals with induced diabetes. These animals with induced diabetes are a model having no abnormality of the prostate (having no benign prostatic hyperplasia). On the basis of the body weight and the fasting blood glucose level, the animals (models) with induced diabetes were grouped or divided into three (3) groups by randomized, multivariable block allocation.

To the three model groups with induced diabetes, a 0.5 v/v% Polysorbate 80 solution (0.5% P) (10 mL/kg), the compound A (3 mg/kg), and the compound A (10 mg/kg) were orally administered, respectively, wherein the administration schedule in all groups was a frequency of once a day, only on weekdays, over four (4) weeks. Moreover, to the above-mentioned first group, a 0.5 v/v% Polysorbate 80 solution (0.5% P) (10 mL/kg) was orally administered in the same administration schedule (normal group). On the last day of administration, a cannula was inserted into and secured to the dome of the bladder under isoflurane anesthesia, and cystometry (Cystometogram, CMG) was conducted in the order of under awake condition and under urethane anesthesia to measure the intravesical pressure, the voided volume, and the residual urine volume. After the completion of the measurement, the blood was collected under deep isoflurane anesthesia, and then the prostate (ventral prostate, dorsolateral prostate), the seminal vesicle, the bladder, and the kidney were extracted and were each weighed. Moreover, the blood glucose level in the collected blood was measured.

The test substances and the dosage in the test system (rats having the urination disorder associated with diabetes) are as follows.

Normal group: citrate buffer 10 mL/kg (intraperitoneal administration), 0.5% P 10 mL/kg (oral administration) (n = 10)
Solvent group (control group): STZ 50 mg/kg (intraperitoneal administration), 0.5% P 10 mL/kg (oral administration) (n = 10)
Group A3: STZ 50 mg/kg (intraperitoneal administration), Compound A 3 mg/kg (oral administration) (n = 10)
Group A10: STZ 50 mg/kg (intraperitoneal administration), Compound A 10 mg/kg (oral administration) (n = 9)

Urination parameter:

The resulting data of the intravesical pressure, the voided volume, and the residual urine volume were analyzed, and the bladder capacity (mL), the residual urine volume (mL), and the urination efficiency (%) were determined as urination parameters in each urination. With respect to these urination parameters, the average value of two measurements was calculated and was taken as a measured value of each individual.

Tissue weight:

From the weights of the ventral prostate and the dorsolateral prostate, the weight of the whole prostate was

calculated. Each weight of the prostate, the seminal vesicle, the bladder, and the kidney was converted to a tissue weight per 100 g of the body weight. For Group A3 and Group A10, which received the compound A, the inhibition rate of each weight of the prostate and the seminal vesicle relative to each weight of the prostate and the seminal vesicle in the solvent group was calculated (with one decimal place).

In statistical analysis, the average value and the standard deviation were calculated for each group with respect to the final body weight, the tissue weights (the prostate, the seminal vesicle, the bladder, and the kidney) per 100 g of the body weight, the blood glucose level, and various urination parameters. For the statistical analysis, significant difference tests were performed with SAS statistical analysis system (SAS version 9.4; manufactured by SAS Institute Japan Ltd.). Two-sided Student's t test was performed between the normal group and the solvent group, and it was judged that a significance level (critical rate) below 5% has a statistically significant difference. Moreover, Williams's multiple comparison test was performed between the solvent group and Group A3 and between the solvent group and Group A10, and it was judged that a significance level (critical rate) below 2.5% has a statistically significant difference.

Asterisk "*" indicates a significance of 5% ($P < 0.05$ (versus the normal group) (Student's t test)), and pound "#" indicates a significance of 2.5% ($P < 0.025$ (versus the solvent group) (Williams's multiple comparison test)).

Bladder contraction force by nomogram analysis:

In cystometry (CMG) under anesthesia, the maximum urinary flow rate (mL/second) and the intravesical pressure ($cmH_2O$) at maximum flow were determined to evaluate the bladder contraction force on urination for each group. Specifically, for each group, the average value of the maximum urinary flow rate Qmax (mL/second), that of the intravesical pressure at maximum flow PdetQmax ($cmH_2O$), and that of the bladder capacity BC (mL) were calculated, and the maximum urinary flow rate/bladder capacity on the Y-axis was plotted relative to the intravesical pressure at maximum flow/bladder capacity on the X-axis. It was judged that the shorter the distance from the origin is, the weaker the bladder contraction force is, and the farther the distance from the origin is, the stronger the bladder contraction force is.

The results are shown in Table 1, Fig. 11 to Fig. 13 (cystometry (CMG) data under awake condition), Fig. 14 to Fig. 16 (cystometry (CMG) data under anesthesia), and Fig. 17 (nomogram analysis results). In each column in Table, "average value ± standard deviation" is listed.

[Table 1]

Table 1

| | | Normal group | Model with induced diabetes | | |
|---|---|---|---|---|---|
| | | | Solvent group | Group A3 | Group A10 |
| Body weight (g) | | 405.3 ± 47.1 | 322.2 ± 43.8* | 327.5 ± 51.3 | 356.4 ± 60.9 |
| Prostate (mg/100g body weight) | Ventral prostate (atrophy rate) | 129.7 ± 28.9 | 109.1 ± 9.7* | 62.2 ± 11.4# (43.0%) | 53.1 ± 13.5# (51.3%) |
| | Dorsolateral prostate (atrophy rate) | 70.2 ± 14.9 | 63.2 ± 13.0 | 35.3 ± 7.8# (44.2%) | 28.8 ± 6.1# (54.5%) |
| | Whole (atrophy rate) | 199.8 ± 41.2 | 172.3 ± 14.3 | 97.5 ± 17.7# (43.4%) | 81.9 ± 19.0# (52.5%) |
| Seminal vesicle (mg/100g body weight) (atrophy rate) | | 85.3 ± 15.7 | 76.1 ± 11.2 | 44.3 ± 9.6# (41.8%) | 37.9 ± 6.4# (50.2%) |
| Bladder (mg/100g body weight) | | 28.1 ± 4.1 | 80.2 ± 12.1* | 79.3 ± 17.5 | 72.9 ± 11.6 |
| Kidney (mg/100g body weight) | | 662.8 ± 68.5 | 1180.3 ± 156.5* | 1114.9 ± 189.2 | 1171.0 ± 213.3 |
| Blood glucose level (mg/dL) | | 247.4 ± 69.1 | 898 ± 371.2* | 912.8 ± 177.3 | 846.7 ± 342.5 |

[Body weight, tissue weight, and blood glucose level]

The solvent group (the control group) showed a significantly decreased body weight and significantly increased bladder and kidney weights in comparison with the normal group. Moreover, the blood glucose level was also significantly increased. In contrast, Group A3 and Group A10 showed significantly decreased prostate and seminal vesicle weights and showed no action or influence on the body weight, the blood glucose level, or the bladder and kidney weights.
As apparent from the blood glucose level of Group A3 and Group A10 relative to the solvent group, no therapeutic effect on diabetes is found in the compound A. Moreover, the model with induced diabetes develops a urination disorder having no enlarged prostate.

[Cystometry (CMG): Bladder capacity, residual urine volume, and urination efficiency]

Fig. 11 to Fig. 13 show the analysis results of the cystometry (CMG) measurement data under awake condition. The solvent group (the control group) showed significantly increased bladder capacity and residual urine volume and a significantly reduced urination efficiency in comparison with the normal group. In comparison with the solvent group (the control group), Group A3 showed significantly reduced bladder capacity and residual urine volume and an improved urination efficiency. Group A10 also showed significantly reduced bladder capacity and residual urine volume and a significantly improved urination efficiency (P = 0.0359, Williams's multiple comparison test).
Fig. 14 to Fig. 16 show the analysis results of the cystometry (CMG) measurement data under anesthesia. The solvent group (the control group) showed significantly increased bladder capacity and residual urine volume and a significantly reduced urination efficiency in comparison with the normal group. In comparison with the solvent group (the control group), Group A3 showed a significantly decreased residual urine volume. Group A10 also showed a significantly decreased residual urine volume and a reduced bladder capacity (P = 0.0336, Williams's multiple comparison test). Moreover, Group A3 and Group A10 showed an improved urination efficiency.
Further, in the nomogram analysis (Fig. 17), it is judged that Group A3 and Group A10, each of which had a farther distance from the origin, have a strong bladder contraction force on urination in comparison with the solvent group (the control group).
Thus, the compound A shows no therapeutic effect on diabetes (Group A3 and Group A10) and further significantly improves the urination disorder in the model with induced diabetes having no enlarged prostate.

Test Example 5

For the compound A and the compound D (mifepristone), the antiprogesterone activity was measured. Specifically, a sex hormone and a test substance were repeatedly administered to ovariectomized rats as a test system to examine the antiprogesterone action (antiprogesterone activity) based on, as an index, the fluctuation of the uterine weight (the inhibitory action relative to the increase of the weight) associated with rat deciduoma formation.
More specifically, rats (female, Crl:CD(SD), 5-week-old: Charles River Laboratories Japan, Inc.) were subjected to ovariectomy, and from one (1) week after the ovariectomy, a high dose estrone of 5 μg/one animal was hypodermically administered to the rats once a day for three (3) days. From the day after one day rest period, the rats received, once a day over eight (8) days, hypodermic administration of a low dose estrone (1 μg/one animal), intramuscular administration of progesterone (6 mg/kg), and oral administration of a test substance. In the middle of the administration, on Day 4 of the start of administration, the rats were subjected to a stimulation treatment of decidualization reaction to the right uterus. The day after the final administration, the uterine weight (right uterine body) was measured (n = 6). Group E was subjected to ovariectomy and a stimulation treatment of decidualization reaction but was not subjected to a hormone treatment.
The test substances and the dosage in the test system (ovariectomized rats) are as follows.

Group E: rats with ovariectomy without hormone treatment
0.5 v/v% Polysorbate 80 solution, 10 mL/kg (n = 6)
Solvent group (control group): 0.5 v/v% Polysorbate 80 solution, 10 mL/kg (n = 6)
Group A3: Compound A, 3 mg/kg (n = 6)
Group A10: Compound A, 10 mg/kg (n = 6)
Group A30: Compound A, 30 mg/kg (n = 6)
Group D: Compound D (mifepristone), 3 mg/kg (n = 6)

The results are shown in Fig. 18.
As shown in Fig. 18, compared with the solvent group, Group D showed a significant inhibitory action (inhibition rate: 89.5%). Compared with the solvent group, although Group A3 did not show a significant inhibitory action (inhibition

rate: 15.0%), Group A10 and Group A30 showed a significant inhibitory action (inhibition rate: 63.0% and 83.3%, respectively). The results have revealed that the compound A has an antiprogesterone activity.

Test Example 6

As described below, a model of a urination disorder associated with streptozocin-induced diabetes was prepared as a urination disorder model in accordance with Test Example 4 to evaluate the effects of the compound A and the compound C on a urination disorder. This model has no benign prostatic hyperplasia and is a model of a urination disorder associated with neuropathy and smooth muscle contraction disorder.

The body weights of Rats (male, Crl:CD(SD), 5-week-old; Charles River Laboratories Japan, Inc.) were measured. On the basis of the body weight, the rats were grouped or divided into two (2) groups. The day after the rats were grouped, a fist group received intraperitoneal administration of a citrate buffer (pH 4.5) (10 mL/kg) having a concentration of 0.1 mmol/L, and a second group received intraperitoneal administration of streptozocin (STZ; Sigma-Aldrich) (50 mg/kg). Streptozocin (STZ) was administered in the form of a solution of streptozocin having a concentration of 5 mg/mL in the citrate buffer.

About one (1) week after streptozocin (STZ) was administered, the body weight and the fasting blood glucose were measured. In the streptozocin (STZ) administration group (the second group), individuals having a fasting blood glucose level of not less than 300 mg/dL were used as animals with induced diabetes. These animals with induced diabetes are a model having no abnormality of the prostate (having no benign prostatic hyperplasia). On the basis of the body weight and the fasting blood glucose level, the animals (models) with induced diabetes were grouped or divided into three (3) groups by randomized, multivariable block allocation.

To the three model groups with induced diabetes, a 0.5 v/v% Polysorbate 80 solution (0.5% P) (10 mL/kg), the compound A (3 mg/kg), and the compound C (dutasteride) (0.3 mg/kg) were orally administered, respectively, wherein the administration schedule in all groups was a frequency of once a day, only on weekdays, over four (4) weeks. Moreover, to the above-mentioned first group, a 0.5 v/v% Polysorbate 80 solution (0.5% P) (10 mL/kg) was orally administered in the same administration schedule (normal group). On the last day of administration, a cannula was inserted into and secured to the dome of the bladder under isoflurane anesthesia, and cystometry (Cystometogram, CMG) was conducted in the order of under awake condition and under urethane anesthesia to measure the intravesical pressure, the voided volume, and the residual urine volume. After the completion of the measurement, the blood was collected under deep isoflurane anesthesia, and then the prostate (ventral prostate, dorsolateral prostate) was extracted and each was weighed. Moreover, the blood glucose level in the collected blood was measured.

The test substances and the dosage in the test system (rats having the urination disorder associated with diabetes) are as follows.

Normal group: citrate buffer 10 mL/kg (intraperitoneal administration), 0.5% P 10 mL/kg (oral administration) (n = 10)
Solvent group (control group): STZ 50 mg/kg (intraperitoneal administration), 0.5% P 10 mL/kg (oral administration) (n = 10)
Group A3: STZ 50 mg/kg (intraperitoneal administration), Compound A 3 mg/kg (oral administration) (n = 10)
Group C0.3: STZ 50 mg/kg (intraperitoneal administration), Compound C (dutasteride) 0.3 mg/kg (oral administration) (n = 10)

Urination parameter: In the same manner as in Test Example 4, the residual urine volume (mL) and the urination efficiency (%) were determined as urination parameters in each urination, and the average value of two measurements was taken as a measured value of each individual.

Prostate weight: In the same manner as in Test Example 4, the weight of the prostate (ventral prostate, dorsolateral prostate, and whole) was converted to a tissue weight per 100 g of the body weight. For Group A3, which received the compound A, and Group C0.3, which received the compound C, the inhibition rate of the weight of the prostate relative to the weight of the prostate in the solvent group was calculated (with one decimal place).

In statistical analysis, significant difference tests were performed in the same manner as Test Example 4. Two-sided Student's t test was performed between the normal group and the solvent group to judge that a significance level (critical rate) below 5% has a statistically significant difference, wherein asterisk "*" indicates a significance of 5%. Moreover, Dunnett's multiple comparison test was performed between the solvent group and Group A3 and between the solvent group and Group C0.3 to judge that a significance level (critical rate) below 5% has a statistically significant difference, wherein pound "#" indicates a significance of 5%.

Bladder contraction force by nomogram analysis: In the same manner as in Test Example 4, each group was evaluated for the bladder contraction force on urination. The maximum urinary flow rate/bladder capacity on the Y-axis was plotted relative to the intravesical pressure at maximum flow/bladder capacity on the X-axis. It was judged that the shorter distances from the origin is, the weaker the bladder contraction force is, and the farther distance from the origin

is, the stronger the bladder contraction force is.

The results are shown in Table 2, Figs. 19 and 20 (cystometry (CMG) data under awake condition) and Fig. 21 (nomogram analysis results).

[Table 2]

Table 2

| | | Normal group | Model with induced diabetes | | |
|---|---|---|---|---|---|
| | | | Solvent group | Group A3 | Group C0.3 |
| Prostate (mg/100g body weight) | Ventral prostate (atrophy rate) | 112.4 ± 32.9 | 101.9 ± 30.3 | 61.2 ± 12.4# (40.0%) | 58.4 ± 8.4# (42.7%) |
| | Dorsolateral prostate (atrophy rate) | 64.3 ± 12.4 | 64.8 ± 16.8 | 38.8 ± 4.2# (40.2%) | 40.5 ± 6.3# (37.5%) |
| | Whole (atrophy rate) | 176.7 ± 40.2 | 166.7 ± 45.8 | 99.9 ± 15.5# (40.1%) | 98.9 ± 12.2# (40.7%) |
| Blood glucose level (mg/dL) | | 261.6 ± 43.3 | 925.3 ± 225.2* | 972.4 ± 312.3 | 984.2 ± 282.0 |

[Prostate weight and blood glucose level]

Compared with the solvent group, Group A3 and Group C0.3 showed significant decrease in the weights of the ventral prostate, the dorsolateral prostate, and the whole prostate with the same level ($P < 0.05$). The comparison of the normal group with the solvent group shows no significant difference in the prostate weight, and thus it is conformed that the model with induced diabetes is a model having no enlarged prostate. For such a diabetes model, since Group A3 and Group C0.3 did not statistically affect the blood glucose level of the solvent group as the same as in Test Example 4 described above, it has confirmed that the compound A and the compound C show no therapeutic effect on diabetes.

[Cystometry (CMG): Residual urine volume and urination efficiency]

From the analysis results of the cystometry (CMG) measurement data under awake condition, as shown in Fig. 19 and Fig. 20, the solvent group showed a significantly increased residual urine volume and a significantly reduced urination efficiency associated with STZ treatment (induction of diabetes) in comparison with the normal group ($p < 0.05$). From these results, the model with induced diabetes develops a urination disorder. Meanwhile, Group A3 showed a significantly decreased residual urine volume in comparison with the solvent group ($p < 0.05$), although Group C0.3 did not statistically affect the residual urine volume in comparison with the solvent group. Moreover, Group A3 showed a significantly improved urination efficiency in comparison with the solvent group ($p < 0.05$), while Group C0.3 did not statistically affect the urination efficiency in comparison with the solvent group. In the urination efficiency, it was confirmed that there was a tendency to show a statistical difference between Group A3 and Group C0.3 ($p = 0.0757$, Student's t test).

In the nomogram analysis (Fig. 21), the normal group showed the farthest distance from the origin, and Group C0.3 was at almost the same position as the solvent group. In contrast, Group A3 showed a farther distance from the origin in comparison with the solvent group. From these results, it is judged that Group A3 has a strong bladder contraction force on urination in comparison with the solvent group and Group C0.3.

Thus, the compound A (Group A3) shows no therapeutic effect on diabetes and further significantly improves the urination disorder even in the model with induced diabetes having no enlarged prostate.

Examples 1 to 7 and Reference Examples 1 to 6 [Discoloration test]

In a mortar, 1 part by mass of the following active ingredient and 99 parts by mass of one of the following carriers were mixed. In a cylindrical screw tube bottle (diameter: 1 cm), 100 mg of the resulting mixture (sample) were placed and stored under the conditions of a temperature of 40°C and a relative humidity of 75% in a constant temperature room. To the initial color difference ($\Delta E^*$) of the mixture, each of the color differences ($\Delta E^*$) of the mixture at a storage

period of two (2) weeks, four (4) weeks, and 6 (six) weeks (for some carriers, 8 (eight) weeks) was evaluated.

For color difference (ΔE*) measurement, an L*a*b* colorimeter ("Spectrophotometer" manufactured by KONICA MINOLTA, INC.) was used. The color difference (ΔE*) was measured by irradiating 100 mg of the sample filled in a cylindrical screw tube bottle (diameter: 1 cm) with light from the bottom of the tube bottle. A color difference (ΔE*) of not less than 3 [an increase in color difference (ΔE*) by not less than 3] at a storage period of four (4) weeks allowed visual confirmation of coloration or discoloration; a color difference (ΔE*) of less than 3 [an increase or decrease in color difference (ΔE*) by less than 3] at a storage period of four (4) weeks was evaluated as no coloration or discoloration.

For carriers, there are cases in which a diluent and/or a disintegrator is classified into a binder and in which a diluent is classified into a disintegrator. In the Examples, the carriers are classified and encoded into a diluent, a disintegrator, and a lubricant as described below for convenience.

[Active ingredient]: 17α-acetoxy-6-chloro-15β-hydroxy-2-oxa-4,6-pregnadiene-3,20-dione

[Diluent]

A-1: Lactose hydrate: "Dilactose S" manufactured by Freund Corporation
A-2: Crystalline cellulose: "CEOLUS UF711" manufactured by Asahi Kasei Corp.
A-3: Corn starch: "Nisshoku Kyokuho Corn Starch" manufactured by NIHON SHOKUHIN KAKO CO., LTD.
A-4: Anhydrous dibasic calcium phosphate: "Fujicalin" manufactured by Fuji Chemical Industries Co., Ltd.

[Disintegrator]

B-1: Pregelatinized starch: "Swelstar PD-1" manufactured by Asahi Kasei Corp.
B-2: Crospovidone: "Kollidon CL" manufactured by BASF
B-3: Low substituted hydroxypropylcellulose: "L-HPC" manufactured by Shin-Etsu Chemical Co., Ltd.
B-4: Carmellose calcium: "E.C.G-505" manufactured by GOTOKU CHEMICAL COMPANY LTD.
B-5: Croscarmellose sodium: "KICCOLATE" manufactured by Asahi Kasei Corp.
B-6: Sodium starch glycolate: "EXPLOTAB" manufactured by JRS PHARMA

[Lubricant]

C-1: Magnesium stearate: "Magnesium stearate" manufactured by TAIHEI CHEMICAL INDUSTRIAL CO., LTD. (magnesium content of magnesium stearate: 4.11% by mass)
C-2: Talc: "Lissbran" manufactured by Kihara Kasei Co., Ltd.
C-3: Stearic acid: "Kolliwax S Fine" manufactured by BASF Japan Ltd.

Table 3 and Fig. 22 to Fig. 23 show the evaluation results of the color difference (ΔE*).

[Table 3]

Table 3 (Color difference: ΔE*)

| | Carrier | 40°C, 75%RH | | | |
|---|---|---|---|---|---|
| | | 2 weeks | 4 weeks | 6 weeks | 8 weeks |
| Example 1 | A-1: Lactose hydrate | 0.26 | 0.90 | 0.45 | - |
| Example 2 | A-2: Crystalline cellulose | 0.26 | 0.26 | 0.19 | - |
| Example 3 | A-3: Corn starch | 0.78 | 1.34 | 1.12 | - |
| Example 4 | A-4: Anhydrous dibasic calcium phosphate | 2.18 | 2.06 | 2.10 | - |

(continued)

| | Carrier | 40°C, 75%RH | | | |
|---|---|---|---|---|---|
| | | 2 weeks | 4 weeks | 6 weeks | 8 weeks |
| Example 5 | B-1: Pregelatinized starch | 0.35 | 0.21 | 0.25 | 0.20 |
| Example 6 | B-2: Crospovidone | 0.86 | 0.93 | 1.10 | 1.21 |
| Example 7 | B-3: Low substituted hydroxypropylcellulose | 0.31 | 1.28 | 1.55 | 1.76 |
| Reference Example 1 | B-4: Carmellose calcium | 2.71 | 3.80 | 4.14 | 4.51 |
| Reference Example 2 | B-5: Croscarmellose sodium | 5.66 | 6.28 | 7.44 | 7.69 |
| Reference Example 3 | B-6: Sodium starch glycolate | 1.61 | 4.68 | 4.38 | 5.26 |
| Reference Example 4 | C-1: Magnesium stearate | 0.26 | 1.05 | 0.87 | - |
| Reference Example 5 | C-2: Talc | 0.22 | 0.15 | 0.46 | - |
| Reference Example 6 | C-3: Stearic acid | 1.26 | 1.31 | 2.01 | - |

As apparent from Table 3, Fig. 22 and Fig. 23, the mixtures (samples) of Examples 1 to 7 are substantially free from a carrier that is a metal salt-form of an organic acid or contain a carrier that is a polyvalent metal salt-form of an inorganic acid, and each have a color difference ΔE* of not more than 3. Thus, the solid preparations (mixtures or samples) of these Examples have stably less or no discoloration or coloration. In Example 4, A-4: anhydrous dibasic calcium phosphate (a polyvalent metal salt of an inorganic acid) shows a slightly large color difference ΔE*. In Reference Examples 1 to 3, the disintegrators, specifically B-4: carmellose calcium, B-5: croscarmellose sodium, and B-6: sodium starch glycolate, each are a metal salt-form of an organic acid, and the disintegrators seem to show a large color difference ΔE* and reduce the stability of the active ingredient. For B-6: sodium starch glycolate, there is not much of difference in color difference ΔE* between a storage period of four (4) weeks and that of 6 (six) weeks; this result seems to be attributed to either experimental error or hardly progressed discoloration at the longer storage period.

In contrast, C-1: magnesium stearate as the lubricant had a tendency to increase a color difference ΔE* in a prolonged storage period. Although this lubricant is a metal salt-form, probably because of high hydrophobicity, the color difference ΔE* is small and the stability of the active ingredient is not damaged. Moreover, C-2: talc showed a small color difference ΔE* over a long period of time. C-3: stearic acid, which is water-insoluble and is a hydrophobic higher saturated fatty acid, had a tendency to increase a color difference ΔE* at the longer storage period, while the color difference ΔE* is small, which shows less or no coloration and less or no damaged stability of the active ingredient.

A combination of the active ingredient with a carrier (a diluent, a disintegrator) that is not a metal salt-form shows a small color difference ΔE* and less or no damaged stability of the active ingredient. In particular, A-2: crystalline cellulose and B-1: pregelatinized starch showed a small color difference ΔE* even in a prolonged storage period of four (4) weeks and did not show a tendency to increase a color difference ΔE*.

Example 8

Each of the samples (A-1 to A-4, B-1 to B-6, C-1) of Examples 1 to 7 and Reference Examples 1 to 4 was stored for thirty-two (32) weeks under the conditions of a temperature of 40°C and a relative humidity of 75% in a constant temperature room, and was evaluated for the change in color difference (ΔE*) from the initial mixture. The results revealed that the carrier (the diluent, the disintegrator) that is not a metal salt-form, including A-1: lactose hydrate, A-2: crystalline cellulose, A-3: corn starch, B-1: pregelatinized starch, B-2: crospovidone, and B-3: low substituted hydroxypropylcellulose, showed a small color difference ΔE* and no discoloration even after the storage of thirty-two (32) weeks. Moreover, even the carrier that is a metal salt-form, such as A-4: anhydrous dibasic calcium phosphate, shows a small color difference ΔE* and less or no impaired stability of the active ingredient without much effect on the coloration or discoloration of the solid preparation probably because the carrier is a polyvalent metal salt of an inorganic acid. Further, although C-1: magnesium stearate is a metal salt-form, probably because of water-insolubility and high hydrophobicity, the color difference ΔE* was small even after the storage of thirty-two (32) weeks.

Example 9

Each one of two carriers (A-1: lactose hydrate, A-2: crystalline cellulose) was added with different amounts to 1 part by mass of the active ingredient and was mixed in a mortar. In a cylindrical screw tube bottle (diameter: 1 cm),

100 mg of the mixture (sample) was put and was stored for eight (8) weeks under the conditions of a temperature of 40°C and a relative humidity of 75% in a constant temperature room. The mixture was evaluated for the change of the color difference (ΔE*) to the initial color difference (ΔE*). The results are shown in Table 4 and Fig. 24 to Fig. 25.

[Table 4]

Table 4

| Carrier | Added amount (parts by mass) | Color difference (ΔE*) |
|---|---|---|
| A-1: Lactose hydrate | 0.3 | 1.52 |
| | 3 | 0.42 |
| | 10 | 0.16 |
| | 18 | 0.14 |
| | 30 | 0.53 |
| | 99 | 0.38 |
| A-2: Crystalline cellulose | 0.3 | 0.64 |
| | 3 | 0.52 |
| | 10 | 0.45 |
| | 30 | 1.22 |
| | 99 | 0.54 |

As apparent form Table 4 and Fig. 24 to Fig. 25, even when each amount of two carriers (A-1: lactose hydrate, A-2: crystalline cellulose) was changed from 0.3 to 99 parts by mass relative to 1 part by mass of the active ingredient, small color difference (ΔE*) and no discoloration were observed for a long period of time after the storage for eight (8) weeks under the conditions of a temperature of 40°C and a relative humidity of 75%.

Example 10

As shown in Table 5, to the active ingredient and the diluent (lactose hydrate, crystalline cellulose) were mixed one disintegrator selected from carmellose, carmellose calcium and croscarmellose sodium as the disintegrator with different mixing amounts (0, 7, 14, 35, 70, 105, 140 mg), each of the resulting mixtures was stored for one (1) week under the conditions of a temperature of 40°C and a relative humidity of 75% in a constant temperature room, and was evaluated for the change of the color difference (ΔE*) to the initial color difference (ΔE*). The results are shown in Table 6 and Fig. 26.

[Table 5]

Table 5

| Component | | Amount per tablet (mq) |
|---|---|---|
| Active ingredient | 17α-Acetoxy-6-chloro-15β-hydroxy-2-oxa-4,6-preqnadiene-3,20-dione | 5.0 |
| Diluent | Lactose hydrate | 90.5 |
| Diluent | Crystalline cellulose | 50.0 |
| Disintegrator | Carmellose (or carmellose calcium or croscarmellose sodium) | 0 to 140.0 |
| Total | | 145.5 to 285.5 |

[Table 6]

Table 6

| Mixing amount (mg) | Disintegrator | | |
|---|---|---|---|
| | Carmellose | Carmellose calcium | Croscarmellose sodium |
| 0 | 0.15 | 0.28 | 0.30 |
| 7 | 0.35 | 0.85 | 0.58 |
| 14 | 0.52 | 0.79 | 1.87 |
| 35 | 0.46 | 0.63 | 1.50 |
| 70 | 1.25 | 1.17 | 2.24 |
| 105 | 0.58 | 1.21 | 2.81 |
| 140 | 0.64 | 1.34 | 2.50 |

As apparent from Fig. 26, for the disintegrator (carmellose calcium and croscarmellose sodium) that is a metal salt of an organic acid, as the metal content has higher, the color difference (ΔE*) tended to be increased. However, carmellose calcium and croscarmellose sodium showed a color difference (ΔE*) of less than 3, specifically 1.34 and 2.50, respectively, even in the mixing amount of 140 mg having the highest metal content; no discoloration or coloration was found by visual observation.

Moreover, comparing the disintegrator (carmellose) that is not a metal salt-form and the disintegrator (carmellose calcium and croscarmellose sodium) that is a metal salt of an organic acid, carmellose, which is not a metal salt-form, showed a smaller color difference (ΔE*) and showed no discoloration over a longer period of time.

[Preparation Examples]

Preparation Example 1

On the basis of the above results, 100 g of the active ingredient, the diluents (1810 g of lactose hydrate, 1000 g of crystalline cellulose, and 300 g of corn starch), the disintegrator (60 g of pregelatinized starch), and the lubricant (30 g of magnesium stearate) were mixed, and the resulting mixture was tableted to produce a tablet (5-mg tablet) containing the components shown in the following Table 7 per tablet.

[Table 7]

Table 7

| Component | | Amount per tablet (mq) |
|---|---|---|
| Active ingredient | 17α-Acetoxy-6-chloro-15β-hydroxy-2-oxa-4,6-preqnadiene-3,20-dione | 5.0 |
| Diluent | Lactose hydrate | 90.5 |
| Diluent | Crystalline cellulose | 50.0 |
| Diluent | Corn starch | 15.0 |
| Disintegrator | Pregelatinized starch | 3.0 |
| Lubricant | Magnesium stearate | 1.5 |
| | Total | 165.0 |

Preparation Example 2

Mixed were 75 g of the active ingredient, the diluents (2790 g of lactose hydrate, 1500 g of crystalline cellulose, and 450 g of corn starch), the disintegrator (90 g of pregelatinized starch), and the lubricant (45 g of magnesium stearate). The resulting mixture was tableted to produce a tablet (2.5-mg tablet) containing the components shown in the following Table 8 per tablet.

[Table 8]

Table 8

| Component | | Amount per tablet (mg) |
|---|---|---|
| Active ingredient | 17a-Acetoxy-6-chloro-15β-hydroxy-2-oxa-4,6-preqnadiene-3,20-dione | 2.5 |
| Diluent | Lactose hydrate | 93.0 |
| Diluent | Crystalline cellulose | 50.0 |
| Diluent | Corn starch | 15.0 |
| Disintegrator | Pregelatinized starch | 3.0 |
| Lubricant | Magnesium stearate | 1.5 |
| | Total | 165.0 |

Preparation Example 3

Mixed were 300 g of the active ingredient, the diluents (2415 g of lactose hydrate, 1290 g of crystalline cellulose, and 450 g of corn starch), the disintegrator (450 g of pregelatinized starch), and the lubricant (45 g of magnesium stearate). The resulting mixture was tableted to produce a tablet (10-mg tablet) containing the components shown in the following Table 9 per tablet.

[Table 9]

Table 9

| Component | | Amount per tablet (mg) |
|---|---|---|
| Active ingredient | 17a-Acetoxy-6-chloro-15β-hydroxy-2-oxa-4,6-preqnadiene-3,20-dione | 10.0 |
| Diluent | Lactose hydrate | 80.5 |
| Diluent | Crystalline cellulose | 43.0 |
| Diluent | Corn starch | 15.0 |
| Disintegrator | Pregelatinized starch | 15.0 |
| Lubricant | Magnesium stearate | 1.5 |
| | Total | 165.0 |

These tablets were stored over four (4) weeks and eight (8) weeks in a constant temperature room under the conditions of a temperature of 40°C and a relative humidity of 75%RH, nevertheless these tablets showed less or no coloration.

INDUSTRIAL APPLICABILITY

According to the present invention, since the solid preparation (or the urination disorder-improving agent) has an increased stability of the 2-oxapregnane compound represented by the formula (1) and effectively improves the urination disorder such as feeling of incomplete emptying, slow stream, urinary incontinence, or frequent urination. Thus, quality of life of human beings (in particular, older persons) suffering from the urination disorder can be improved and enhanced.

## Claims

1. A solid preparation at least containing (a) an active ingredient and (b) a carrier,

   wherein the active ingredient (a) contains a 2-oxapregnane compound represented by the following formula (1) or a pharmaceutically acceptable salt thereof:

   [Chem. 1]

   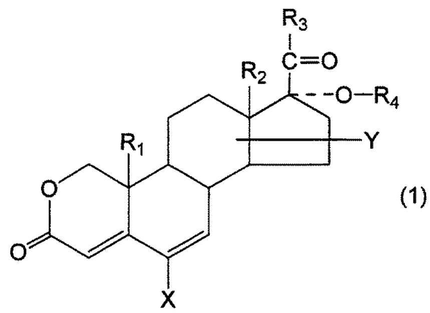

   (1)

   wherein $R_1$ to $R_3$ are the same or different and each represent an alkyl group, $R_4$ represents a hydrogen atom or an alkylcarbonyl group, X represents a halogen atom, and Y represents a hydroxyl group or oxo group bonded to a 11-position, 15-position, or 16-position of a steroid skeleton of the compound, and
   the carrier (b) contains a first carrier and/or a second carrier,
   wherein the first carrier is not a metal salt-form, and the second carrier is a polyvalent metal salt-form of an inorganic acid.

2. The solid preparation according to claim 1, wherein, in the formula (1), $R_1$ to $R_3$ each represent a methyl group, $R_4$ represents an acetyl group, X represents a chlorine atom, and Y represents a hydroxyl group or oxo group bonded to the 15-position of the steroid skeleton of the compound.

3. The solid preparation according to claim 1 or 2, wherein the compound represented by the formula (1) is 17α-acetoxy-6-chloro-15β-hydroxy-2-oxa-4,6-pregnadiene-3,20-dione.

4. The solid preparation according to any one of claims 1 to 3, wherein the carrier (b) contains at least one member selected from (b-1) a diluent, (b-2) a binder, and (b-3) a disintegrator.

5. The solid preparation according to claim 4, wherein the diluent (b-1) is at least one member selected from a saccharide or a sugar alcohol, a starch, a polysaccharide, and an alkaline earth metal salt of an inorganic acid.

6. The solid preparation according to claim 4 or 5, wherein the diluent (b-1) is at least one member selected from a lactose, a corn starch, a crystalline cellulose, and a calcium phosphate, and the disintegrator (b-3) is at least one member selected from a pregelatinized starch, a crospovidone, and a low substituted hydroxypropylcellulose.

7. The solid preparation according to any one of claims 1 to 6, which further contains a lubricant.

8. The solid preparation according to any one of claims 1 to 7, which is a solid preparation for improving a urination disorder or symptom.

9. The solid preparation according to any one of claims 1 to 8, which improves at least one urination disorder or symptom selected from feeling of incomplete emptying, slow stream, hesitancy, straining, terminal dribble, post micturition dribble, urinary incontinence, frequent urination, urgency, bladder pain or bladder pain syndrome, pain on urination, cystatrophia, neurogenic bladder, detrusor overactivity or overactive bladder, chronic cystitis, interstitial cystitis, chronic prostatitis, pelvic pain syndrome, underactive bladder, and detrusor hyperactivity with impaired contractile function.

10. The solid preparation according to any one of claims 1 to 9, which improves at least one urination disorder or symptom selected from residual urine volume, urination efficiency, bladder capacity, bladder wet weight, and kidney wet weight.

11. The solid preparation according to any one of claims 8 to 10, wherein the urination disorder is a urination disorder having no benign prostatic hyperplasia.

12. The solid preparation according to any one of claims 8 to 11, wherein the urination disorder is a urination disorder associated with a smooth muscle contraction disorder.

13. The solid preparation according to any one of claims 8 to 12, wherein the urination disorder is a urination disorder associated with at least one disease selected from the following: a disease causing neurogenic bladder; diabetes; and neuropathy associated with diabetes.

14. The solid preparation according to any one of claims 1 to 13, which improves or promotes urination.

15. The solid preparation according to any one of claims 1 to 14, wherein a dosage of the solid preparation is 0.01 to 100 mg per day in terms of the compound represented by the formula (1).

[Fig. 1]

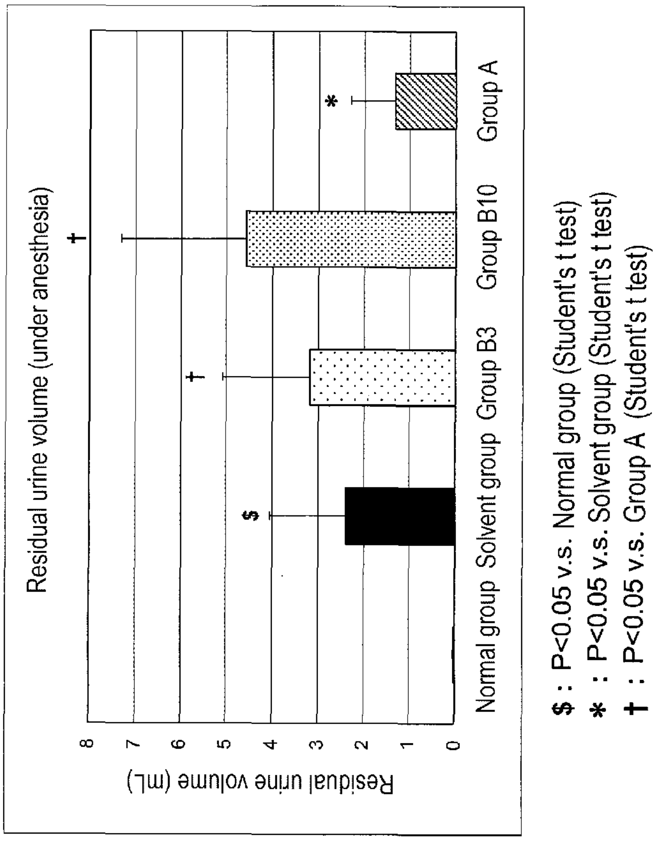
Residual urine volume (under anesthesia)
Residual urine volume (mL)
0
1
2
3
4
5
6
7
8
Normal group
Solvent group
Group B3
Group B10
Group A
$
†
†
*
$ : P<0.05 v.s. Normal group (Student's t test)
* : P<0.05 v.s. Solvent group (Student's t test)
† : P<0.05 v.s. Group A (Student's t test)

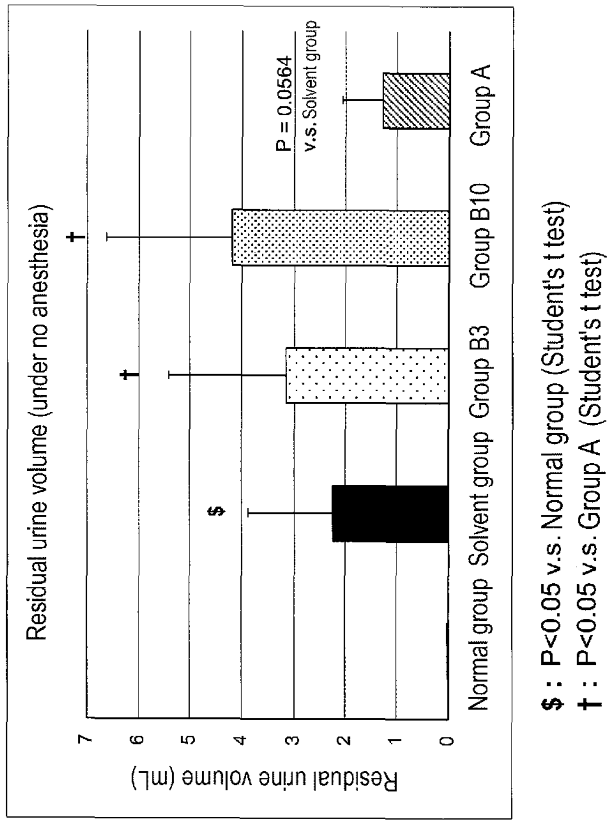
Residual urine volume (under no anesthesia)
Residual urine volume (mL)
0
1
2
3
4
5
6
7
Normal group
Solvent group
Group B3
Group B10
Group A
$
†
†
P = 0.0564
v.s. Solvent group
$ : P<0.05 v.s. Normal group (Student's t test)
† : P<0.05 v.s. Group A (Student's t test)

[Fig. 2]

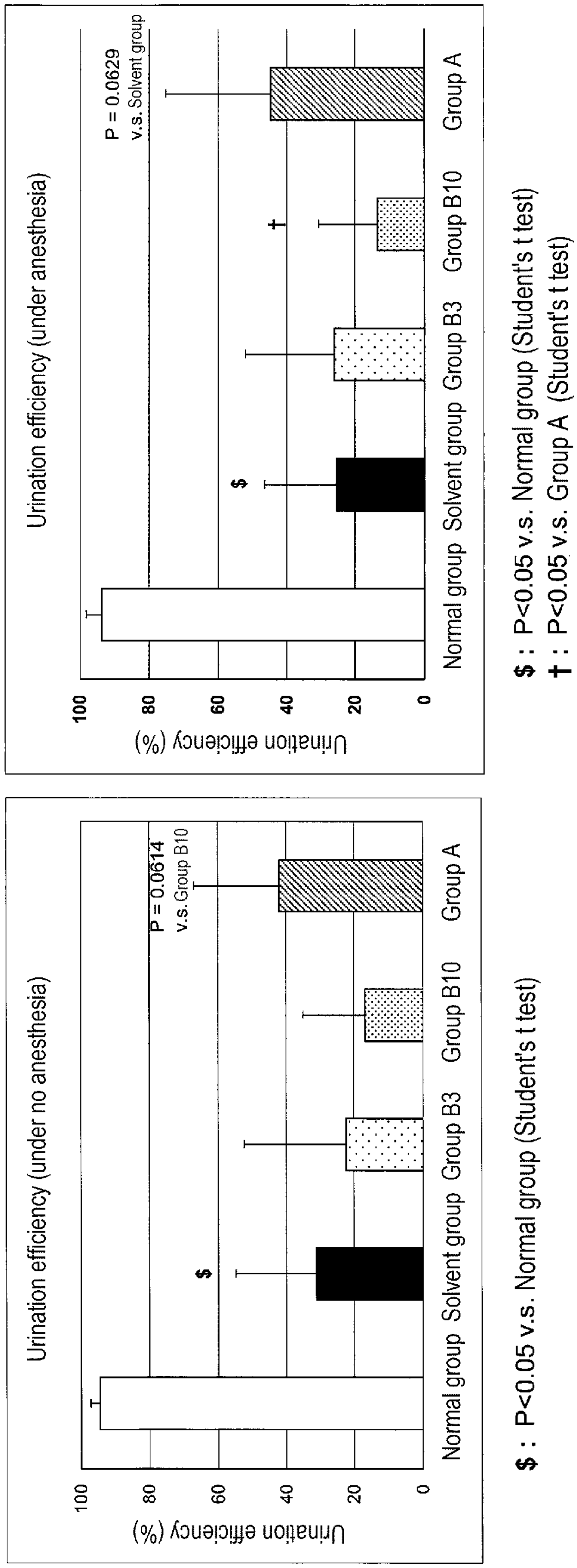
Urination efficiency (under anesthesia)
Urination efficiency (%)
100
80
60
40
20
0
Normal group
Solvent group
Group B3
Group B10
Group A
P = 0.0629
v.s. Solvent group
$
†
$ : P<0.05 v.s. Normal group (Student's t test)
† : P<0.05 v.s. Group A (Student's t test)
Urination efficiency (under no anesthesia)
Urination efficiency (%)
100
80
60
40
20
0
Normal group
Solvent group
Group B3
Group B10
Group A
P = 0.0614
v.s. Group B10
$
$ : P<0.05 v.s. Normal group (Student's t test)

[Fig. 3]

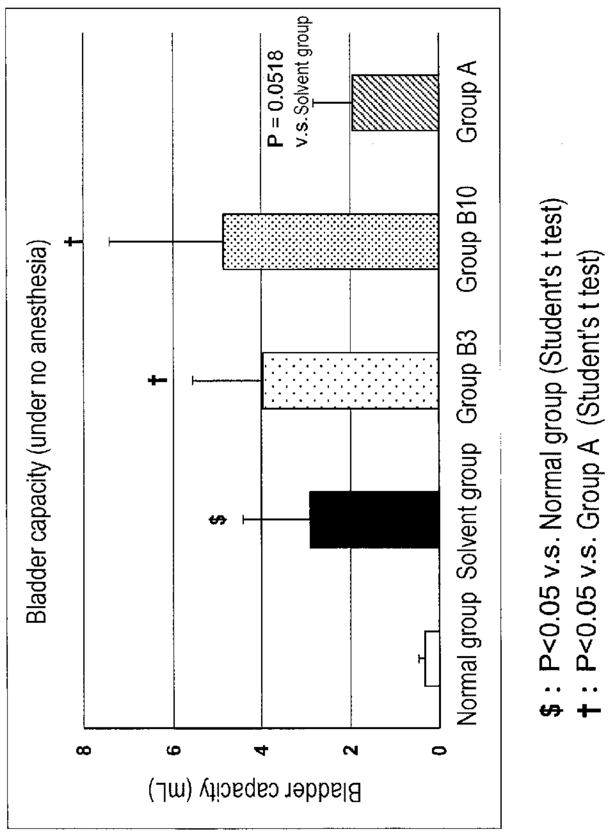
Bladder capacity (under no anesthesia)
Bladder capacity (mL)
0
2
4
6
8
Normal group
Solvent group
Group B3
Group B10
Group A
$
†
†
P = 0.0518
v.s. Solvent group
$ : P<0.05 v.s. Normal group (Student's t test)
† : P<0.05 v.s. Group A (Student's t test)

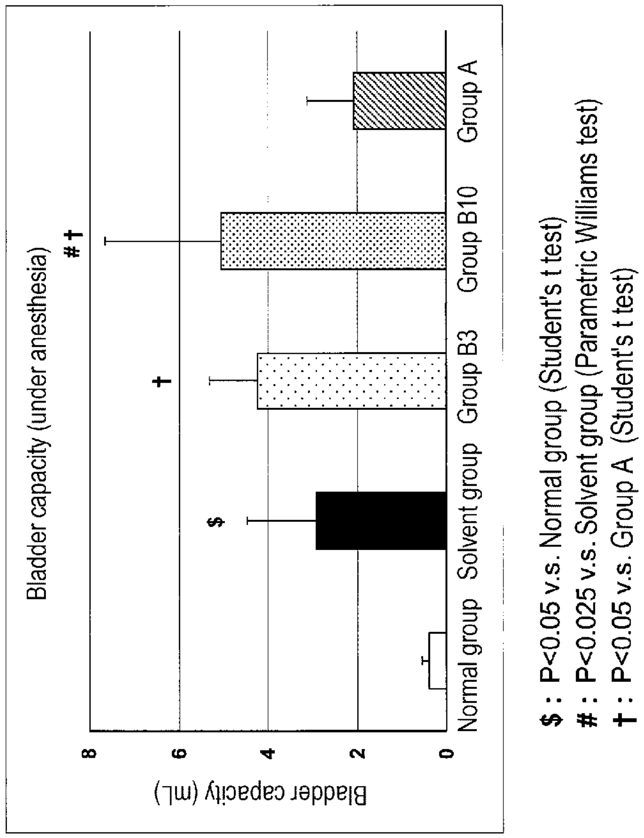
Bladder capacity (under anesthesia)
Bladder capacity (mL)
0
2
4
6
8
Normal group
Solvent group
Group B3
Group B10
Group A
$
†
# †
$ : P<0.05 v.s. Normal group (Student's t test)
# : P<0.025 v.s. Solvent group (Parametric Williams test)
† : P<0.05 v.s. Group A (Student's t test)

[Fig. 4]

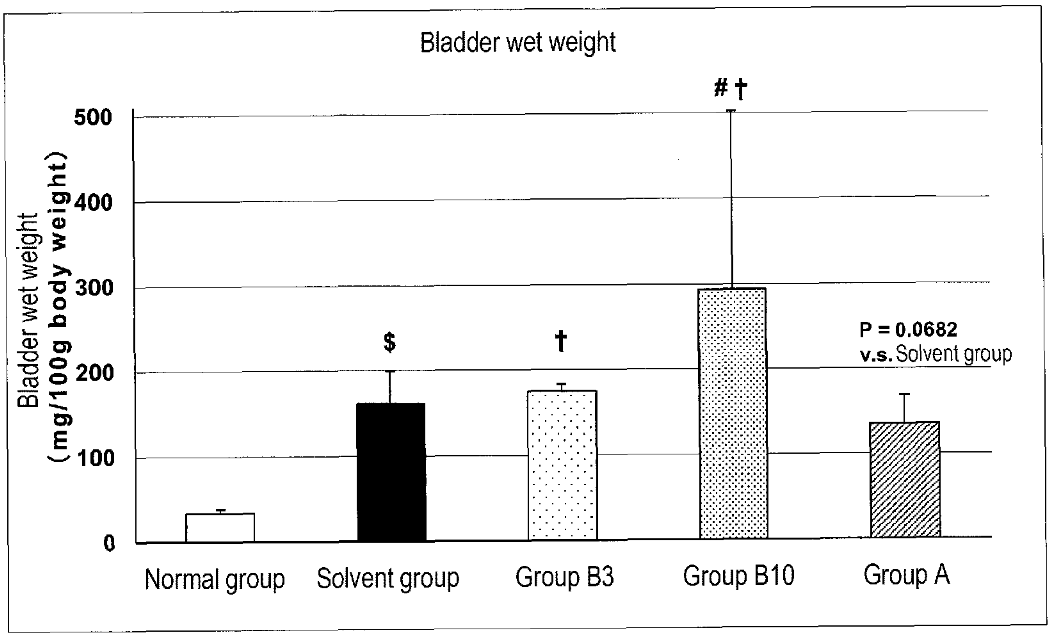


$ : P<0.05 v.s. Normal group (Student's t test)
# : P<0.025 v.s. Solvent group (Parametric Williams test)
† : P<0.05 v.s. Group A (Student's t test)

[Fig. 5]

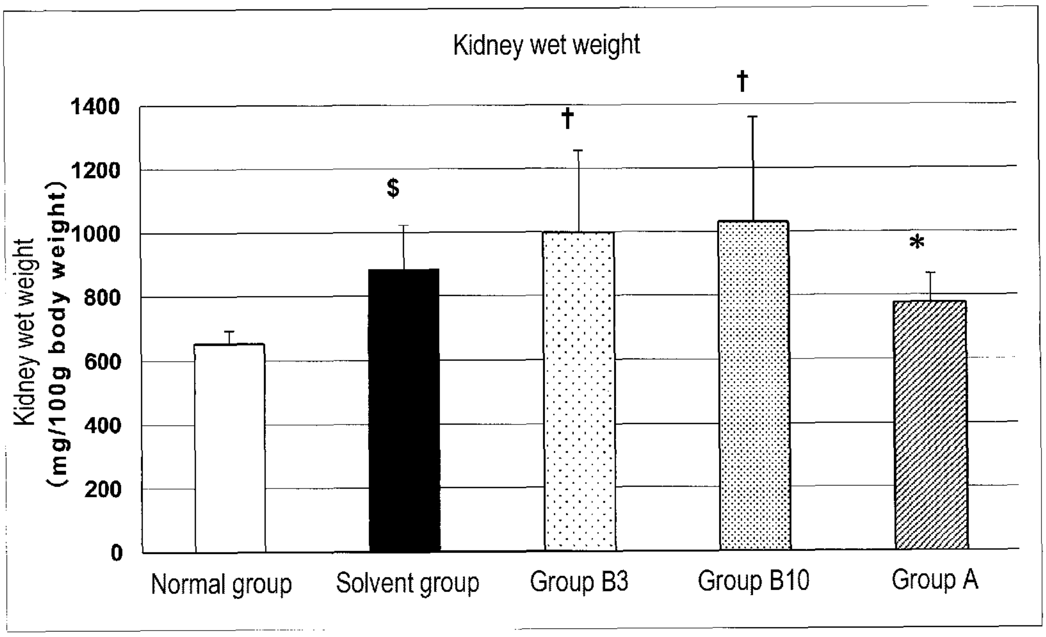


$ : P<0.05 v.s. Normal group (Student's t test)
* : P<0.05 v.s. Solvent group (Student's t test)
† : P<0.05 v.s. Group A (Student's t test)

[Fig. 6]

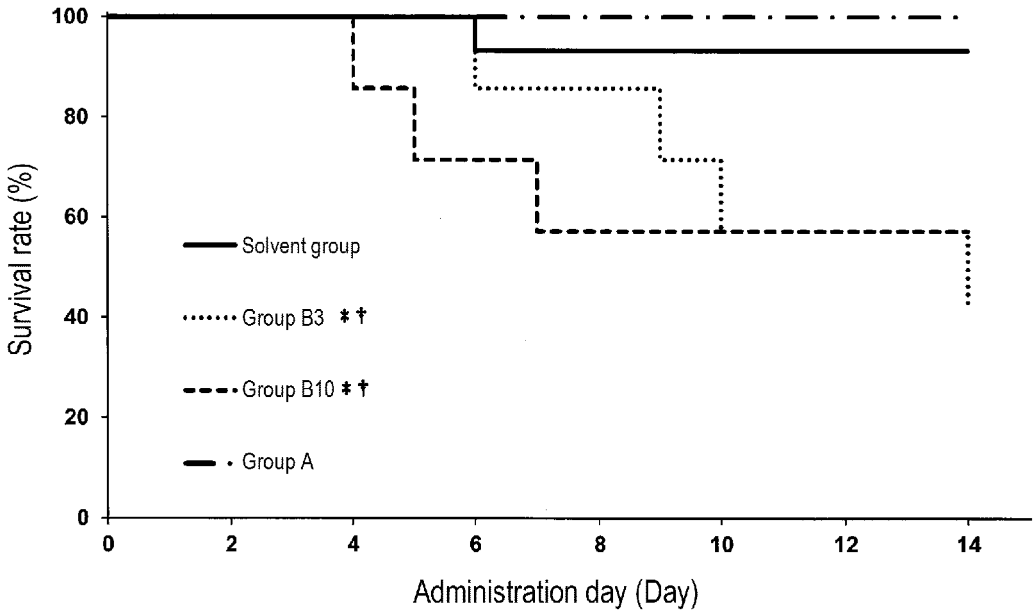


* : P<0.05 vs Solvent group (Log-rank test)
† : P<0.05 vs Group A (Log-rank test)

[Fig. 7]

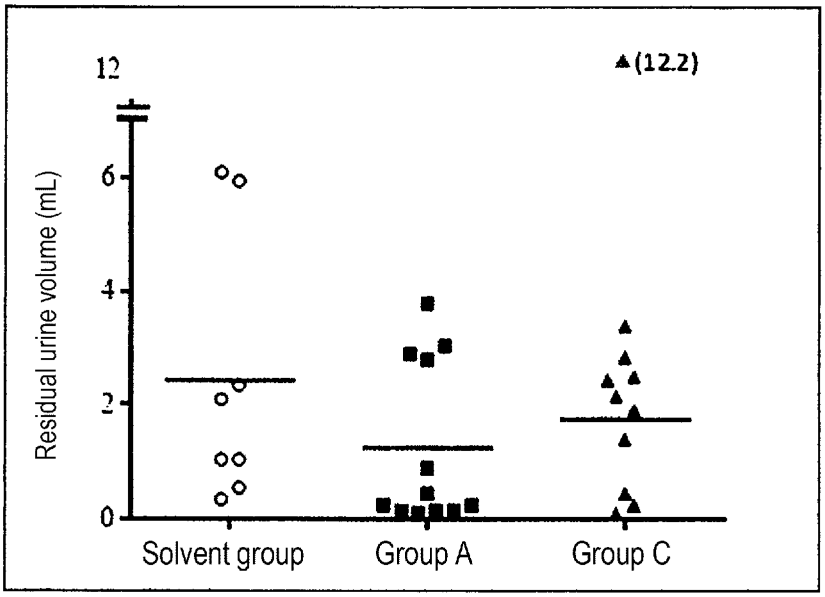

[Fig. 8]

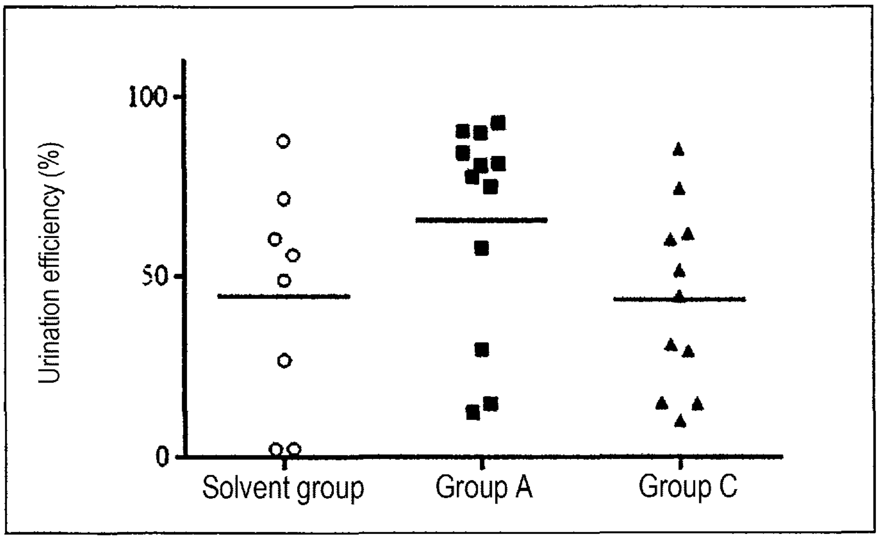


[Fig. 9]

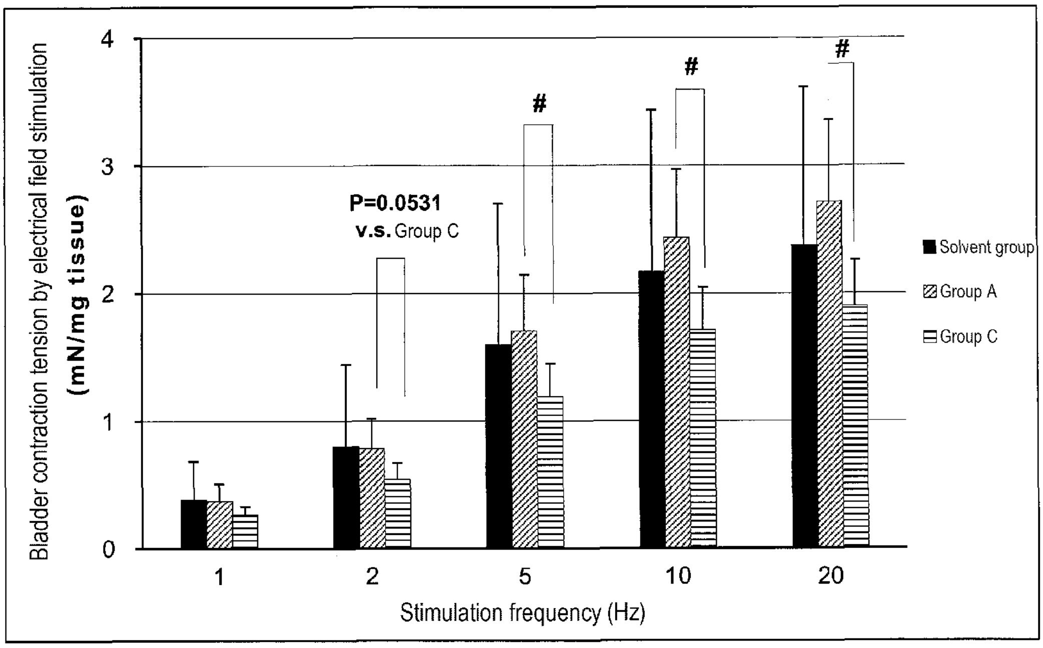


#: P<0.05 v.s. Group C (Student's t test)

[Fig. 10]

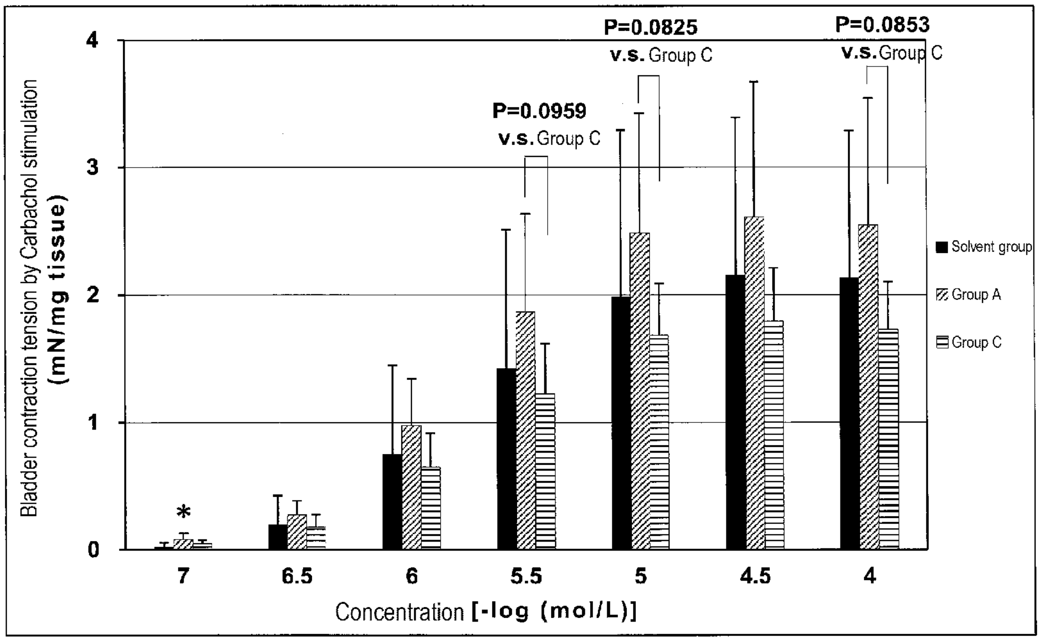
P=0.0825
v.s. Group C
P=0.0853
v.s. Group C
P=0.0959
v.s. Group C
Bladder contraction tension by Carbachol stimulation
(mN/mg tissue)
4
3
2
1
0
*
7
6.5
6
5.5
5
4.5
4
Concentration [-log (mol/L)]
Solvent group
Group A
Group C

[Fig. 11]

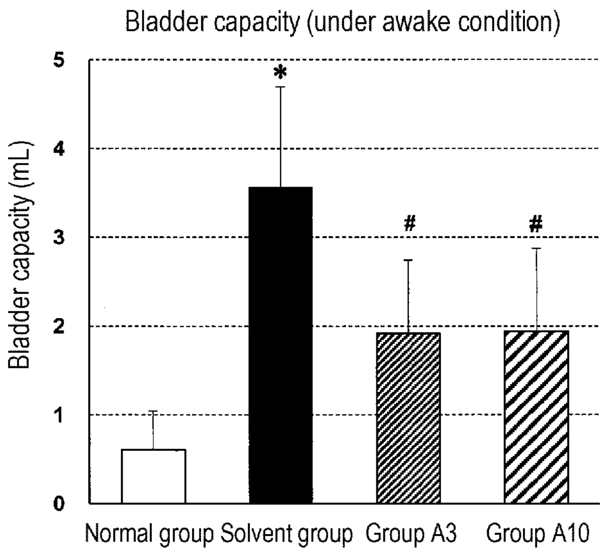
Bladder capacity (under awake condition)
Bladder capacity (mL)
5
4
3
2
1
0
*
#
#
Normal group
Solvent group
Group A3
Group A10

[Fig. 12]

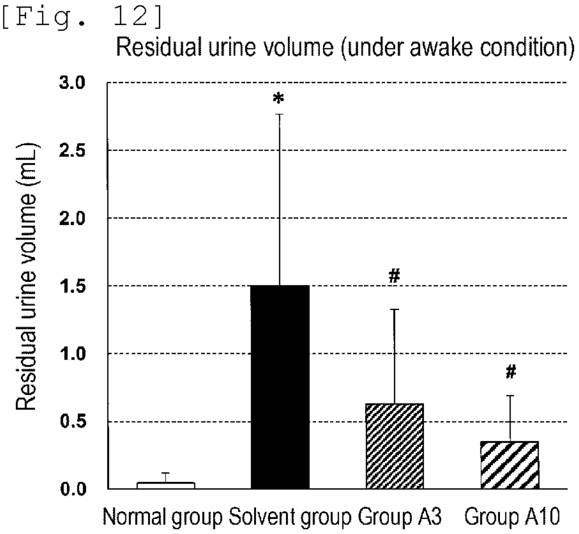


[Fig. 13]

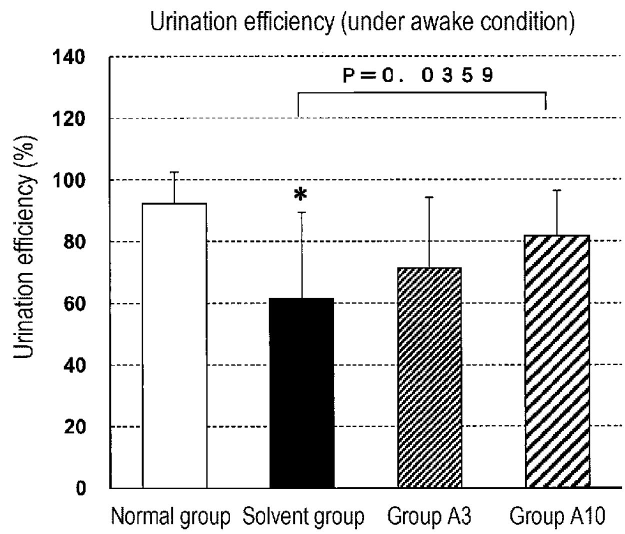


[Fig. 14]

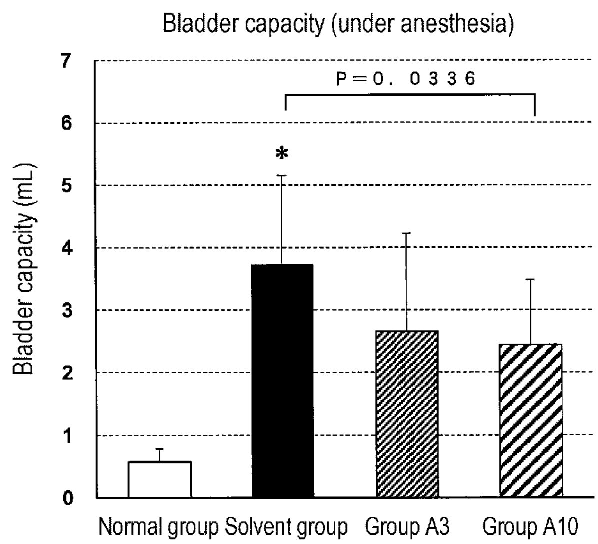

[Fig. 15]

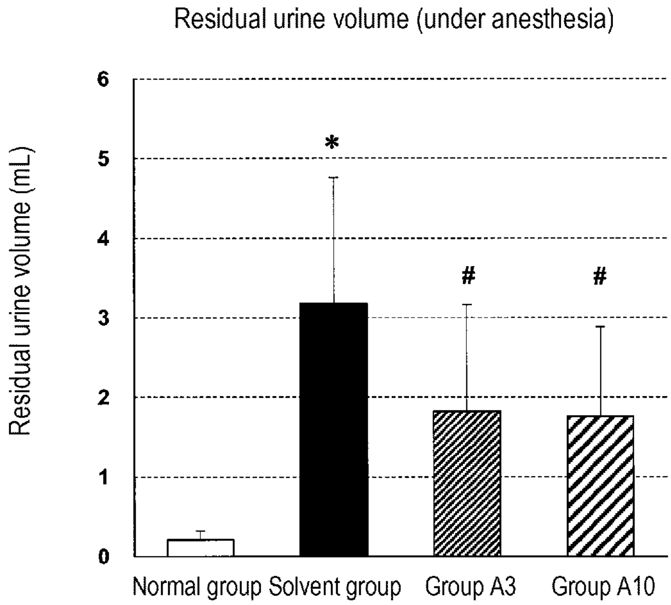
Residual urine volume (under anesthesia)
Residual urine volume (mL)
0
1
2
3
4
5
6
*
#
#
Normal group
Solvent group
Group A3
Group A10

[Fig. 16]

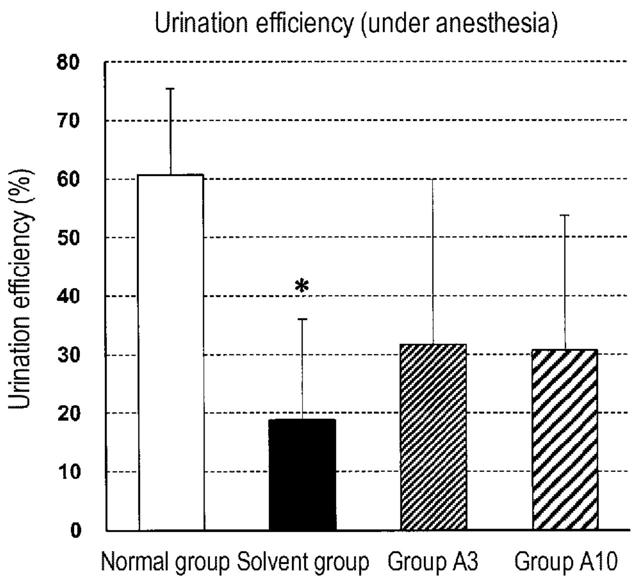
Urination efficiency (under anesthesia)
Urination efficiency (%)
0
10
20
30
40
50
60
70
80
*
Normal group
Solvent group
Group A3
Group A10

[Fig. 17]

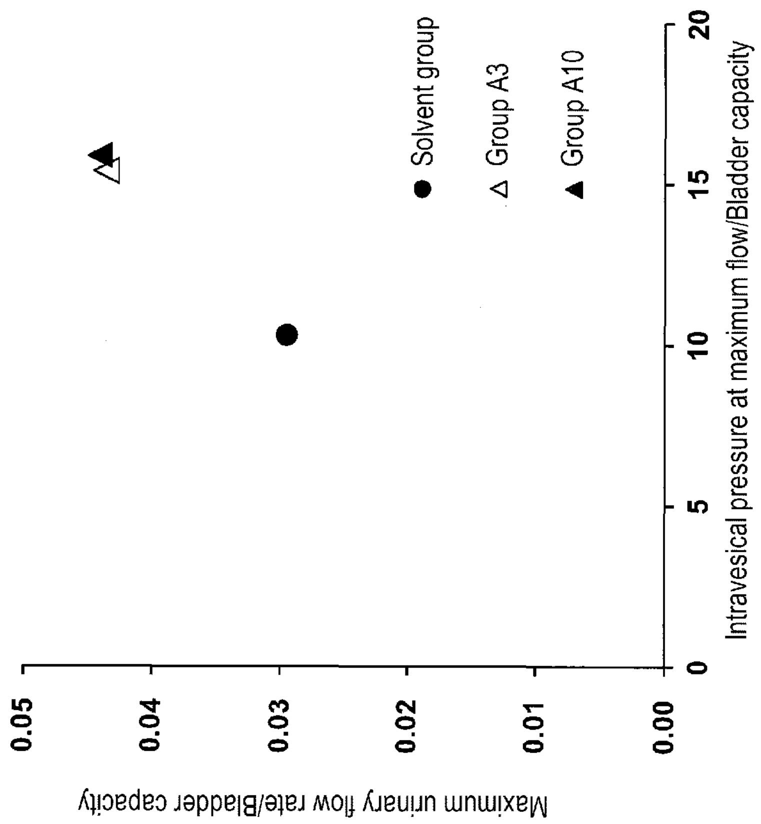
Maximum urinary flow rate/Bladder capacity
0.05
0.04
0.03
0.02
0.01
0.00
Intravesical pressure at maximum flow/Bladder capacity
0
5
10
15
20
Solvent group
Group A3
Group A10

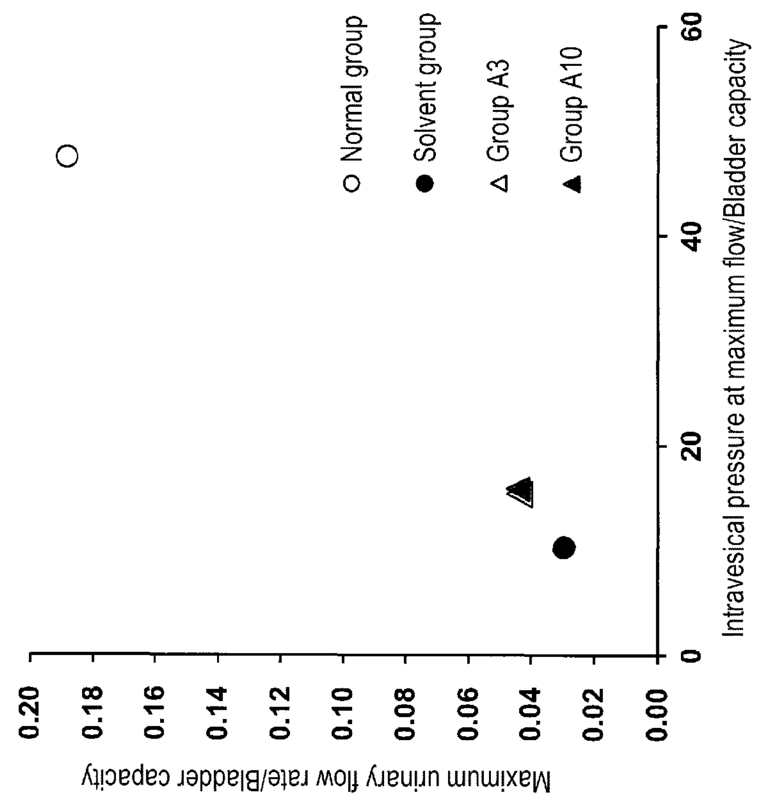
Maximum urinary flow rate/Bladder capacity
0.20
0.18
0.16
0.14
0.12
0.10
0.08
0.06
0.04
0.02
0.00
Intravesical pressure at maximum flow/Bladder capacity
0
20
40
60
Normal group
Solvent group
Group A3
Group A10

[Fig. 18]

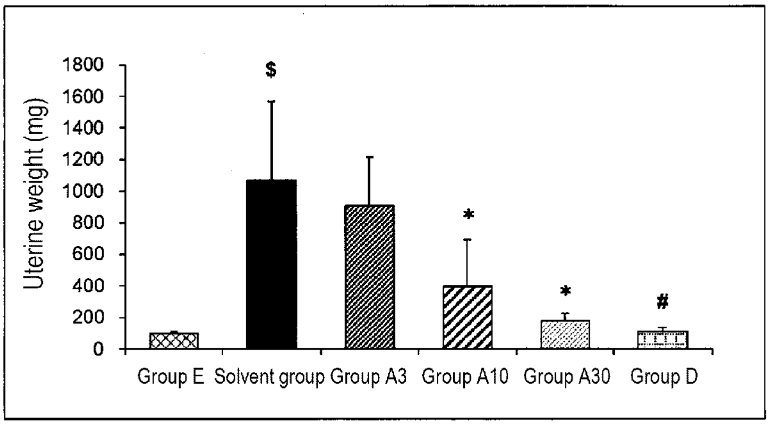


$ : P < 0.05 vs Group E (Student's t test)

* : P< 0.025 vs Solvent group (Williams's multiple comparison test)

# : P < 0.05 vs Solvent group (Student's t test)

[Fig. 19]

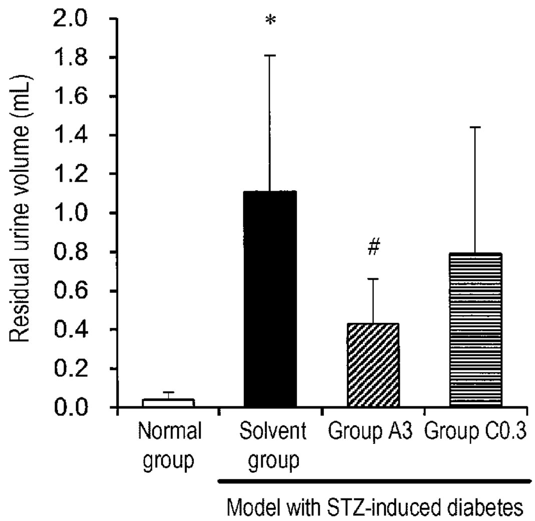


[Fig. 20]

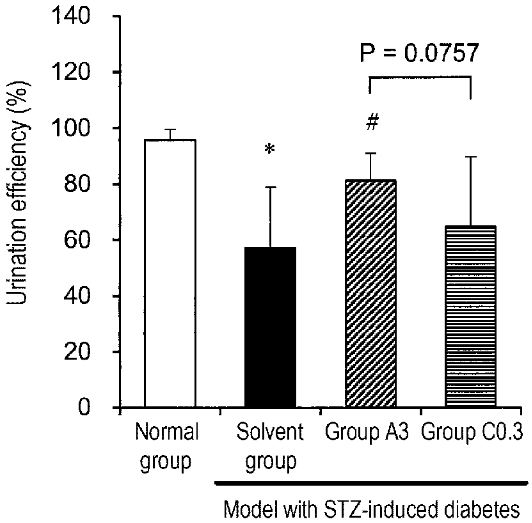

[Fig. 21]

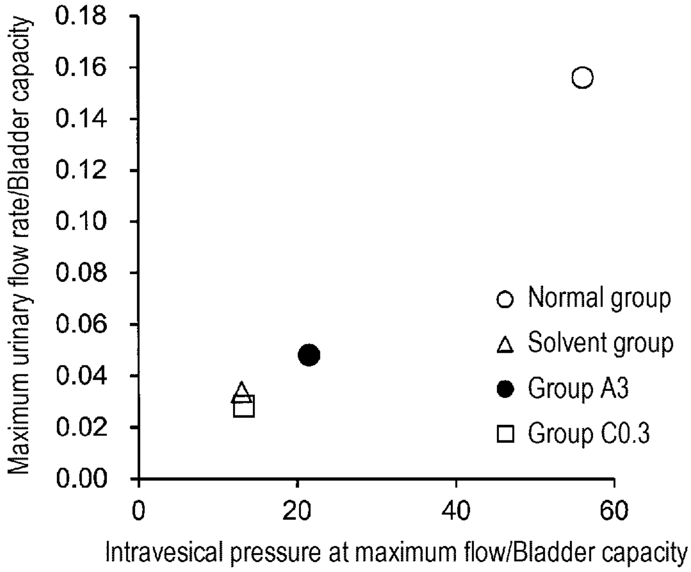
Maximum urinary flow rate/Bladder capacity
0.18
0.16
0.14
0.12
0.10
0.08
0.06
0.04
0.02
0.00
0
20
40
60
Intravesical pressure at maximum flow/Bladder capacity
Normal group
Solvent group
Group A3
Group C0.3

[Fig. 22]

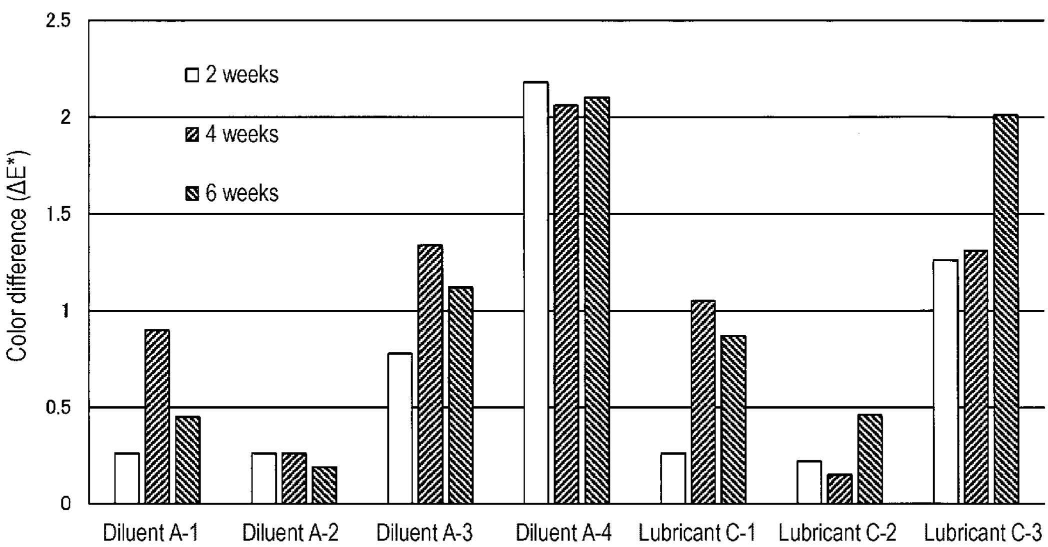
Color difference (ΔE*)
2.5
2
1.5
1
0.5
0
2 weeks
4 weeks
6 weeks
Diluent A-1
Diluent A-2
Diluent A-3
Diluent A-4
Lubricant C-1
Lubricant C-2
Lubricant C-3

[Fig. 23]

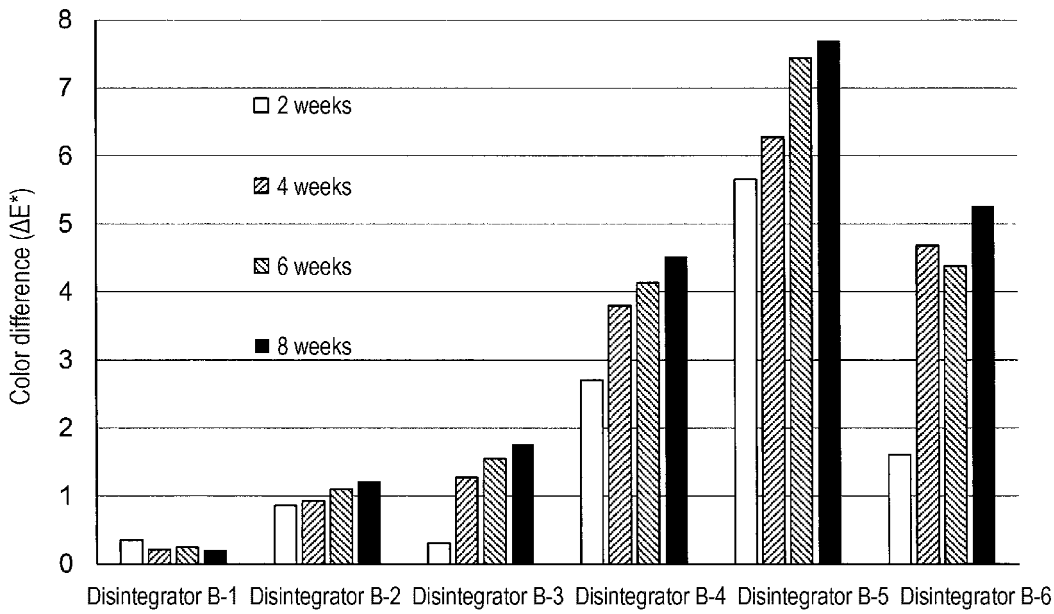
Color difference (ΔE*)
8
7
6
5
4
3
2
1
0
2 weeks
4 weeks
6 weeks
8 weeks
Disintegrator B-1
Disintegrator B-2
Disintegrator B-3
Disintegrator B-4
Disintegrator B-5
Disintegrator B-6

[Fig. 24]

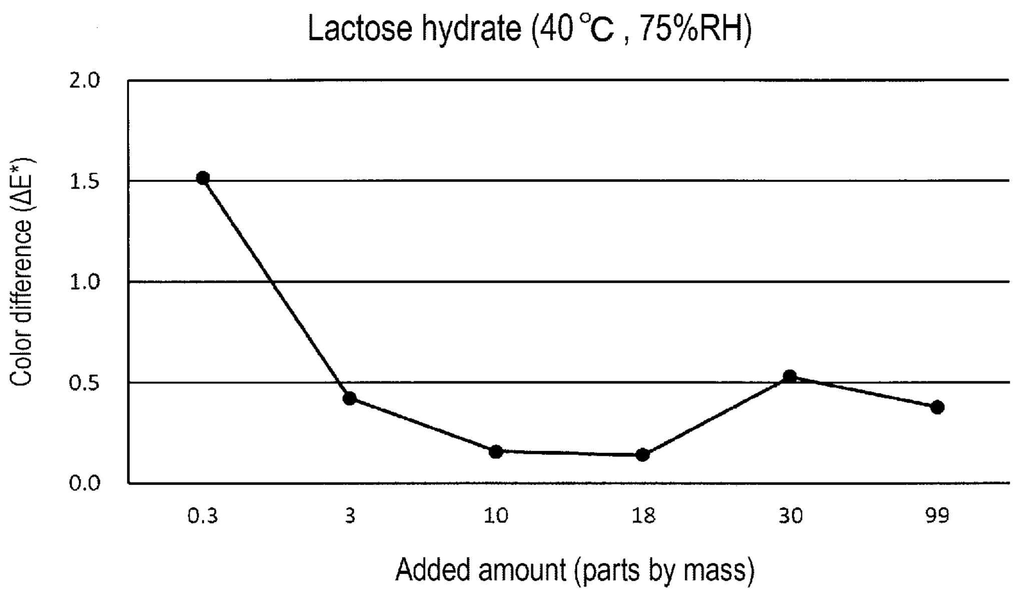
Lactose hydrate (40℃, 75%RH)
Color difference (ΔE*)
2.0
1.5
1.0
0.5
0.0
0.3
3
10
18
30
99
Added amount (parts by mass)

[Fig. 25]

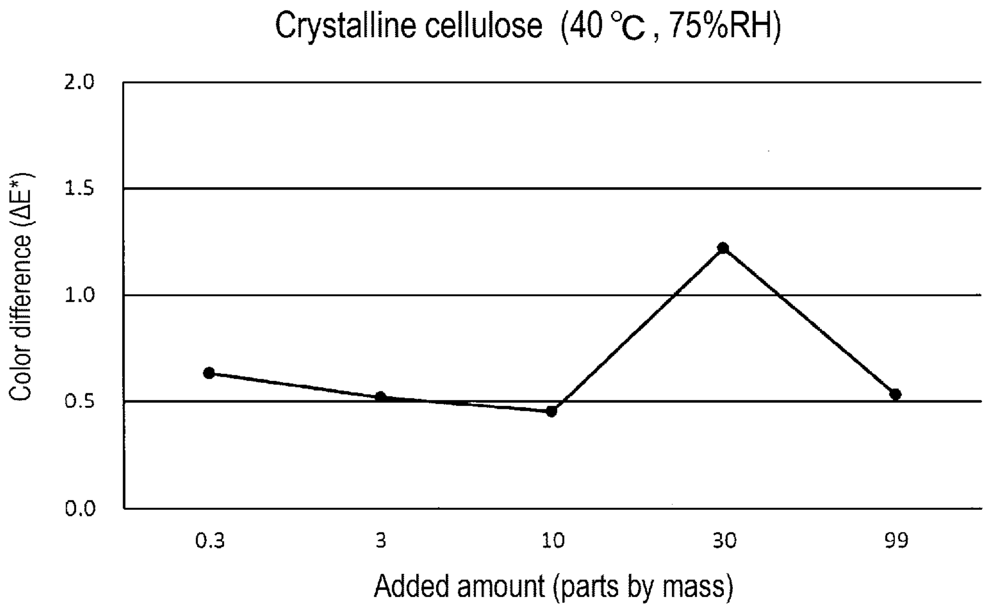
Crystalline cellulose (40 °C , 75%RH)
Color difference (ΔE*)
2.0
1.5
1.0
0.5
0.0
0.3
3
10
30
99
Added amount (parts by mass)

[Fig. 26]

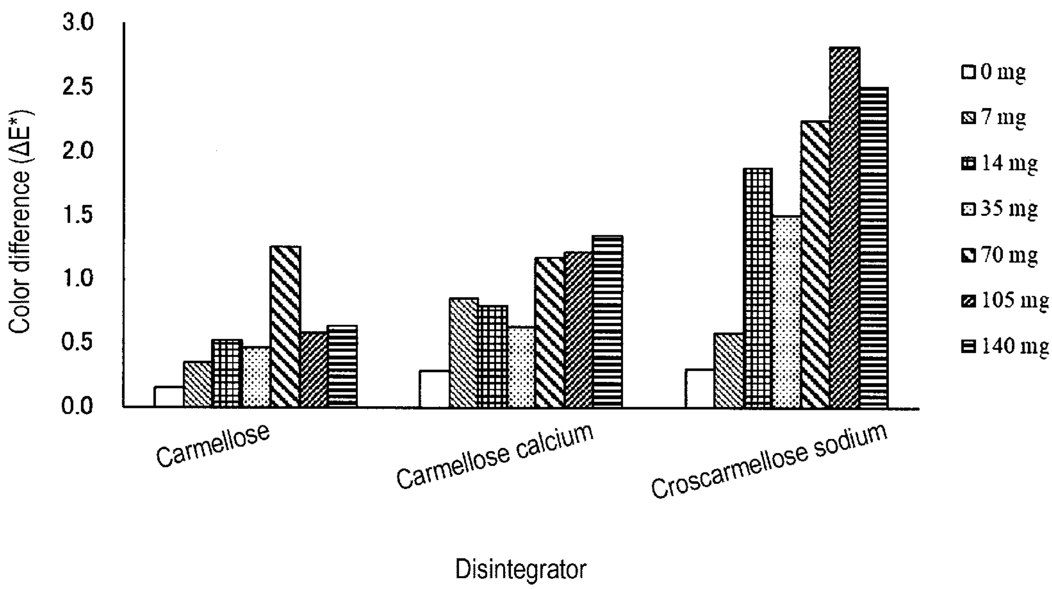
Color difference (ΔE*)
3.0
2.5
2.0
1.5
1.0
0.5
0.0
0 mg
7 mg
14 mg
35 mg
70 mg
105 mg
140 mg
Carmellose
Carmellose calcium
Croscarmellose sodium
Disintegrator

## INTERNATIONAL SEARCH REPORT

International application No.
**PCT/JP2021/046665**

### A. CLASSIFICATION OF SUBJECT MATTER

***A61K 31/585***(2006.01)i; ***A61K 9/20***(2006.01)i; ***A61K 47/10***(2006.01)i; ***A61K 47/26***(2006.01)i; ***A61K 47/32***(2006.01)i; ***A61K 47/36***(2006.01)i; ***A61K 47/38***(2006.01)i; ***A61P 7/10***(2006.01)i; ***A61P 13/02***(2006.01)i; ***A61P 13/10***(2006.01)i
FI: A61K31/585; A61K9/20; A61K47/10; A61K47/26; A61K47/32; A61K47/36; A61K47/38; A61P7/10; A61P13/02; A61P13/10

According to International Patent Classification (IPC) or to both national classification and IPC

### B. FIELDS SEARCHED

Minimum documentation searched (classification system followed by classification symbols)

A61K31/585; A61K9/20; A61K47/10; A61K47/26; A61K47/32; A61K47/36; A61K47/38; A61P7/10; A61P13/02; A61P13/10

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY/MEDLINE/EMBASE/BIOSIS (STN), JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | JP 1-199993 A (TEIKOKU HORMONE MFG CO LTD) 11 August 1989 (1989-08-11) claims, p. 2, upper left column, p. 5, upper left column-lower left column, example 3(f) | 1-7, 15 |
| Y | | 8-10, 14 |
| A | | 11-13 |
| X | WO 2017/195804 A1 (ASKA PHARM CO LTD) 16 November 2017 (2017-11-16) claims, paragraphs [0044]-[0050], [0054] | 1-7, 15 |
| Y | | 8-10, 14 |
| A | | 11-13 |
| Y | 朴英哲ほか, 前立腺肥大症に対するTZP-4238の臨床的検討－尿流動態におよぼす影響－, 泌尿器科紀要, 1994, vol. 40, pp. 761-769 table 4, (PARK, Young-Chol et al. Clinical Study of TZP-4238 on Patients with Benign Prostatic Hypertrophy -with Special Reference to Urodynamics. Acta Urologica Japonica.) | 8-10, 14 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 January 2022** | **01 February 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/046665**

**C. DOCUMENTS CONSIDERED TO BE RELEVANT**

| Category* | Citation of document, with indication, where appropriate, of the relevant passages | Relevant to claim No. |
|---|---|---|
| P, X | WO 2021/065027 A1 (ASKA PHARM CO LTD) 08 April 2021 (2021-04-08) claims, paragraphs [0044]-[0047] | 1-15 |

Form PCT/ISA/210 (second sheet) (January 2015)

**INTERNATIONAL SEARCH REPORT**
**Information on patent family members**

International application No.
**PCT/JP2021/046665**

| Patent document cited in search report | Publication date (day/month/year) | Patent family member(s) | Publication date (day/month/year) |
|---|---|---|---|
| JP 1-199993 A | 11 August 1989 | (Family: none) | |
| WO 2017/195804 A1 | 16 November 2017 | EP 3456729 A1<br>claims, paragraphs [0042]-[0048], [0052]<br>US 2019/0127417 A1<br>CA 3021179 A<br>AU 2017264187 A<br>CN 109153701 A<br>KR 10-2019-0004787 A<br>BR 112018071950 A<br>EA 201892566 A<br>MX 2018013786 A | |
| WO 2021/065027 A1 | 08 April 2021 | JP 2021-59539 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H5155773 A
- JP H83045 A
- WO 2012020749 A
- JP S61204198 A
- JP 2591640 B
- JP 2011020972 A


### Non-patent literature cited in the description


- *Endocrine Journal,* 1994, vol. 41 (4), 445-452
- *World Journal of Urology,* 1995, vol. 13, 9-13
- *The Journal of Urology,* August 1993, vol. 150, 351-358
- The Japanese Pharmacopoeia. Handbook of Pharmaceutical Excipients
- *International Pharmaceutical Excipients Council Japan,* 2007
- Japanese Pharmaceutical Excipients Dictionary 2016. Yakuji Nippo Ltd, February 2016
- Pharmaceutics. Nankodo, Co., Ltd, 1997
- Japanese Pharmaceutical Excipients 2018. Yakuji Nippo Ltd, July 2018
- *Pharmacology,* 1995, vol. 50 (3), 192-200